# EUROPEAN PATENT SPECIFICATION

(11) **EP 2 423 213 B1**
(45) Date of publication and mention of the grant of the patent: **08.01.2014**
(21) Application number: 10766578.8
(22) Date of filing: 09.04.2010
(51) Int. Cl.: C07D 495/04, C07D 335/06, C07D 335/04, A61K 31/4365, A61K 31/382, A61K 9/06, A61K 9/08, A61K 9/02, A61K 9/107, A61K 9/20, A61K 9/48, A61K 9/00, A61P 31/04, A61P 31/10, A61P 35/00

(54) **PREPARATION AND USE OF NOVEL ANTIBIOTIC, ANTICANCER COMPOUNDS AND DERIVATIVES THEREOF**
ZUBEREITUNG UND VERWENDUNG NEUER ANTIBIOTISCHER UND ANTITUMORVERBINDUNGEN SOWIE DERIVATEN DARAUS
PRÉPARATION ET UTILISATION DE NOUVEAUX COMPOSÉS ANTIBIOTIQUES ET ANTICANCÉREUX ET DE LEURS DÉRIVÉS

(30) Priority: 23.04.2009 CN 200910137455; 06.06.2009 CN 200910203152
(43) Date of publication of application: 29.02.2012
(73) Proprietor: Yang, Gengliang, Baoding Hebei 071002 (CN)
(72) Inventor: MA, Zhengyue, Hebei 071002 (CN); TIAN, Wei, Hebei 071002 (CN); FANG, Baoling, Hebei 071002 (CN); WANG, Ge, Hebei 071002 (CN); LI, Linbo, Hebei 071002 (CN); YANG, Chunliu, Hebei 071002 (CN); BAI, Ligai, Hebei 071002 (CN)
(74) Representative: Plucker, Guy
(86) International application number: PCT/CN2010/000471
(87) International publication number: WO 2010/121487

(56) References cited:
- SUN YIFENG PAN WEN-LONG ZHANG DONG-DI ET AL: "Synthesis of Bis-heterocyclic derivatives of 6-Isopropyl-3-formylchromone", JIL INDAXUE ZIRAN KEXUEXUEBAO - ACTA SCIENTIARUM NATURALIUMUNIVERSITATIS JILINENSIS, JILIN DAXUE, BEIJING, CN, vol. 46, no. 5, 1 September 2007 (2007-09-01), pages 45-53, XP008156585, ISSN: 0529-6579
- SUN YIFENG ET AL.: 'Synthesis ofBis-heterocyclic Derivatives of 6-Isopropyl-3-formylchromone.' ACTA SCIENTIARUM NATURALIUM UNIVERSITATIS SUNYATSENI vol. 46, no. 5, September 2007, XP008156585

## Description

### FIELDOF THE INVENTION

The invention relates to the novel antibacterial and anticancer compounds and their derivatives, especially involving the preparation and antibacterial, anti-cancer application of these new anti-bacterial, anti-cancer compounds and their derivatives.

### BACKGROUND OF THE INVENTION

Fungi is one kind of eukaryotic organisms having the similar structures as well as physiological processes with the host cell. Fungi infection is divided into superficial fungal infection and deep fungal infection based on its different site of infection. Superficial fungus infection is mainly caused by various dermatophytes such as hand-foot tinea, porrigo and tinea corporis and usually treated by griseofulvin, nystatin and ketoconazole in the clinical. Deep fungus infection caused by candida albicans, cryptococcus neoformans, cspergillus, mucor, etc. is of great harm and even life-threatening. Moreover, owing to the abuse of broad-spectrum antibiotics, hormone and immunosuppressant, the harmless fungi in organism may cause illness. In recent years, the incidence of fungal infection has obviously increased.

Considering the biological characteristics of fungi, the common antifungal drugs would cause damage to the host cell while it destroyed the fungal cells. Moreover, along with the continual emergence of resistant strains, the treatment of the various illness caused by fungal infection has plunged into a dilemma. Presently, few drugs can be used to treat fungal infection in clinical effectively. Therefore, the development and exploration of new compounds with high antifungal activity is still desired.

Cancer is mainly caused by the chemical, physical and biological (fungal toxins, viruses, etc.) carcinogenic factors. Based on the different parts in body, cancer can be divided into esophageal cancer, lung cancer, breast cancer, liver cancer, etc. Although numerous studies on anti-cancer drugs have been conducted and a series of anticancer drugs such as cisplatin, vinblastine, vincristine, paclitaxel, camptothecin and their derivatives have been found, good activity and non-toxicity of broad-spectrum or narrow spectrum anti-cancer drugs are still not appeared. Therefore, to explore and develop new broad-spectrum and narrow-spectrum active anti-cancer compounds with good activity and low toxicity for the dual effect of treatment and prevention is still a focus of study now.

Cell death is a common phenomenon in the biosphere and myriads of cell die in normal human body everyday in two main ways: necrosis and programmed cell death. Cell necrosis is a passive response for the foreign injury, such as ischemia, fever, chemical and physical damage, biological attacks, that can result in rapid cell death. The main morphological feature of necrosis is cell swelling, which leads to cell membrane rupture and at last its dissolution. Moreover inflammatory cytokines which cause severe inflammation can be released from the cell. Cell necrosis is associated with many kinds of human diseases, such as acute fulminant hepatitis caused by virus infection. Programmed cell death is another way different from cell necrosis controlled by gene. Programmed cell death induced by many factors, including external factors such as radiation, drugs and virus infections, and in vivo factors, such as cancer, autoimmune diseases and other degenerative diseases. It is known that liver cancer, colon cancer, lung cancer, lymphoma, breast cancer, prostate cancer, ovarian cancer and chronic leukemia and so on associated with apoptosis. Therefore, to find an efficient and low toxicity new compound with treatment and prevention of induced programmed cell death and cell death blocked is still urgent work.

It is know in an article publichied in Acta Scientiarum Naturalium universitatis Sunyatseni (Vol. 46, N°. 5, 1 September 2007, pp 49-53) the synthesis of Bis-heterocyclic derivatives of 6-isopropyl-3-formylchromone. However this article does not mention or suggest any antibiotic or anticancer properties for the compounds.

### SUMMARY OF THE INVENTION

The present invention is to provide new anti-bacterial, anti-cancer compounds and their derivatives, stereoisomers, the racemic or non-racemic mixture of stereoisomers, in addition the pharmaceutically acceptable salt or solvate that can be shown by the general formula of I, II, III and IV. 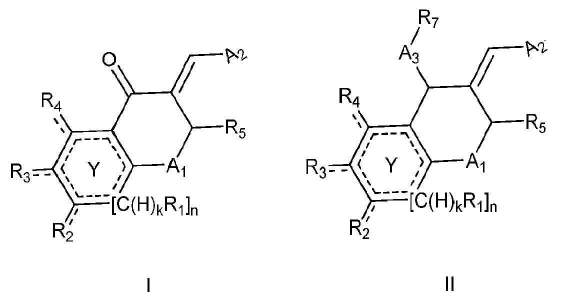 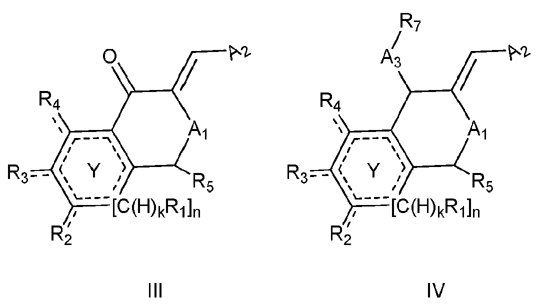 is
Y represents the elements of benzene ring at any position, and it can independently selected from C, O, S and N; when Y is stand for O or S, it is bivalent elements; Y is trivalent elements when it stands for N; and is quadrivalent elements when it stands for C. Y represents the priority to C, N and S.
The dotted lines means the bonds are dispensable. When a bond is double bond, its neighbor bonds are not double bonds.
k is an integer 0 or 1; n is an integer 0,1 or 2
R₁, R₂, R₃ and R₄ can be independently selected from hydrogen, fluorine, chlorine, bromine, iodine, hydroxyl, cyano, C₁₋₂₀-alkyl, C₁₋₂₀-alkyl-oxy, C₁₋₂₀-alkyl carbonyl, or C₁₋₂₀-alkyl-carbonyl-oxy. On condition that at least one of R₁, R₂, R₃ and R₄ is not hydrogen and, the alkyl portion in these groups can be replaced by one or more of the independent halogen atoms such as fluorine, chlorine, bromine and iodine.
R₅ represents hydrogen, C₁₋₂₀-alkyl, C₁₋₂₀-alkyl-oxy, C₁₋₂₀-alkyl-carbonyl, and C₁₋₂₀-alkyl-carbonyl-oxy.
R₆ and R₇ represent hydrogen, alkyl, aryl, substituted aryl, or heteroaryl;
A₁ represents CH₂, CH₂CH₂, O, S, S(O), S(O)₂, or NR₁;
A₂ represents Cl, Br, F or I;
A₃ represents O, S, S(O), S(O)₂, NR, Cl, Br, F, I, or P; when A₃ is Cl, Br, F or I, R₇ does not exist. Among them, the C₁₋₂₀-alkyl can be aromatic alkyl or non-aromatic hydrocarbon, straight-chain alkyl or branched-chain alkyl, cyclic alkyl or non-cyclic alkyl, selected from the priority of C₁₋₂₀-alkyl, C₂₋₂₀-alkenyl, C₂₋₂₀-alkynyl, C₃₋₂₀-cycloalkyl, C₃₋₂₀- cycloalkenyl, C₆₋₂₀-aryl, C₆₋₁₀-aryl-C₁₋₁₀-alkyl, C₃₋₁₀-cycloalkyl-C₁₋₁₀-alkyl, C₃₋₁₀- cycloalkenyl-C₁₋₁₀-alkyl, and C₁₋₁₀-alkyl-C₆₋₁₀-aryl. The more priority is selected from C₁₋₁₀-alkyl, C₂₋₁₀-alkenyl, C₂₋₁₀-alkynyl, C₃₋₁₀-cycloalkyl, C₃₋₁₀-cycloalkenyl, C₆₋₁₀-aryl, C₆₋₁₀-aryl-C₁₋₆-alkyl, C₃₋₆-cycloalkyl-C₁₋₆-alkyl, C₃₋₆-cycloalkenyl-C₁₋₆-alkyl and C₁₋₆-alkyl-C₆₋₁₀-aryl. The most priority is selected from the C₁₋₆-alkyl, C₂₋₆-alkenyl, C₂₋₆-alkynyl, C₃₋₆-cycloalkyl, C₃₋₆-cycloalkenyl, C₆₋₈-aryl, C₆₋₁₀-aryl-C₁₋₃-alkyl, C₃₋₆-cycloalkyl-C₁₋₃-alkyl, C₃₋₆-cycloalkenyl-C₁₋₃-alkyl and C₁₋₃-alkyl- C₆₋₁₀-aryl.

According to some embodiments of the invention, in the new anti-bacterial, anti-cancer compounds and their derivatives represented by the formula of I, II, III and IV, Y can be independently selected from C, O, S and N; K can be independently selected from the integer of 0 and 1; n can independently selected from the integer of 0, 1 and 2; the dotted lines means the bonds are dispensable; when a bond is double bond, its neighbor bonds are not double bonds.

In the new anti-bacterial, anti-cancer compounds and their derivatives that are represented by the formula of I, II, III and IV, R₁, R₂, R₃ and R₄ can optionally be substituted by hydrogen, fluorine, chlorine, bromine, iodine, hydroxyl, methyl, ethyl, n-propyl, isopropyl, n-butyl, isobutyl, amyl, isoamyl, n-hexyl, heptyl, octyl, 2-ethyl-hexyl, vinyl, propenyl, butenyl, pentenyl, ethynyl, propynyl, butynyl, cyclopropyl, cyclohexyl, phenyl, benzyl, naphthyl, naphthylmethyl, methoxyl, ethyoxyl, propoxy, isopropoxy, butoxy, isobutoxy, pentyloxy, inohexyloxy, benzyloxy, trifluoromethyl, 1,1,1-trifluoro ethyl, or 4-fluorine phenyl.

In the new anti-bacterial, anti-cancer compounds and their derivatives that are represented by the general formula of I, II, III and IV, R₅ can be optionally substituted by acetyl, n-propionyl, iso-propionyl, n-butyl acyl, isobutyryl, n-valeryl, isovaleryl, n-hexanoyl, iso-hexanoyl, caprylyl, 2-ethyl-acetyl, nonaneoyl, decanoyl, dodecyl acyl, palmitoyl, linolicacyl, stearinacyl, cycolpropane acyl, hexamethylene acyl, benzoyl, phenylacetyl, naphthoyl, naphthoacetyl, triflyl, 1,1,1-trifluoroacetyl, benzene propionyl, furoyl, and thiozale acyl. When there are alkyls contained in the above groups, the alkyl parts can optionally substituted by one or more independent halogens, such as F, Cl, Br and I.

In the new anti-bacterial, anti-cancer compounds and their derivatives which are represented by the formula of I, II, III and IV, R₇ can be optionally substituted by hydrogen, methyl, ethyl, n-propyl, isopropyl, n-butyl, isobutyl, n-pentyl, isopentyl, n-hexyl, heptyl, octyl, 2-ethyl-hexyl, vinyl, allyl, butenyl, pentenyl, ethinyl, 2-propynyl, 2-butynyl, cyclopropyl, cyclohexyl, phenyl, substituted phenyl, benzyl, naphthyl, naphthal, methoxyl, thyoxyl, propoxyl, isopropoxyl, butoxyl, isobutoxyl, pentyloxyl, hexyliloxy, benzoxy, trifluoromethyl, 1,1,1- trifluoroethyl, furyl, furfuryl, thienyl, 3-methyl-thienyl, pyrryl, pyridyl, 3-methyl-pyridyl, and pyranyl.

In the new anti-bacterial, anti-cancer compounds and their derivatives that are represented by the formula I, II, III and IV, A₁ can be optionally substituted by CH₂, CH₂CH₂, O, S, S(O), S(O)₂, NR1; A₂ can be optionally substituted by Cl, Br, F , I , P; A₃ can be optionally substituted by O, S, S(O), S(O)₂, NR, Cl, Br, F, I, P; when A₃ is Cl, Br, F or I, R₇ does not exist.

In some embodiments of the invention, the compound of formula I is the stereoisomer or mixture of compounds Ia and Ib.

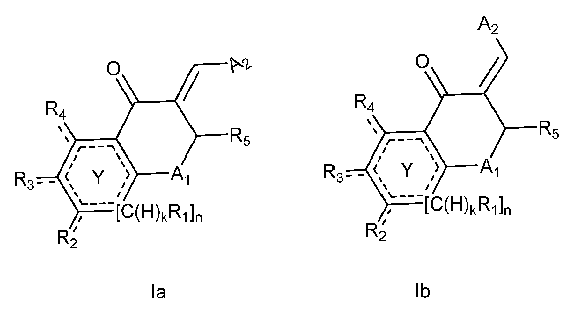

In some embodiments of the invention, the compound of formula II is the stereoisomer or mixture of the compounds IIa and IIb.

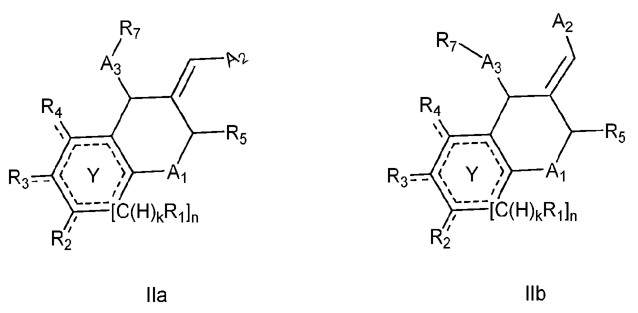

In some embodiments of the invention, the compound of formula III is the stereoisomer or mixture of the compounds IIIa and IIIb.

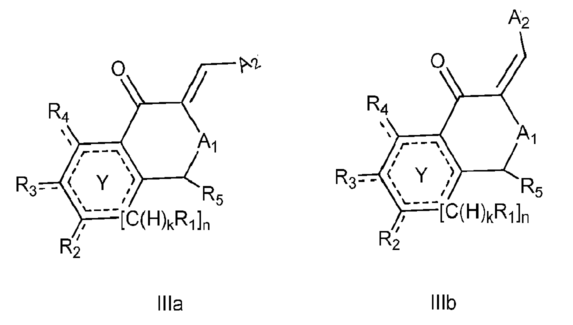

In some embodiments of the invention, the compound of formula IV is the stereoisomer or mixture of the compounds IVa and IVb.

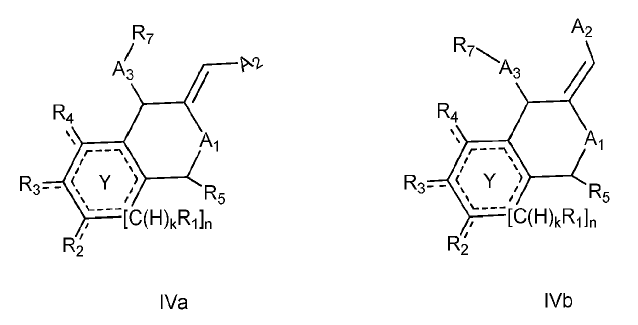

The definition of Y, A₁, A₂, R₁, R₂, R₃, R₄, R₅, R₇ are the same as above.

Another aspect of the invention is to provide a new method to synthesize the anti-bacterial, anti-cancer compounds and their derivatives, stereoisomers, the racemic or non-racemic mixture of stereoisomers, and the pharmaceutically acceptable salt or solvate that can be included by the general formula I, II, III and IV
1) The compounds VIII are prepared by the reaction of compounds V with compound VII and Base 1; The compounds IX are prepared by the reaction of compounds VT with compound VII and Base 1 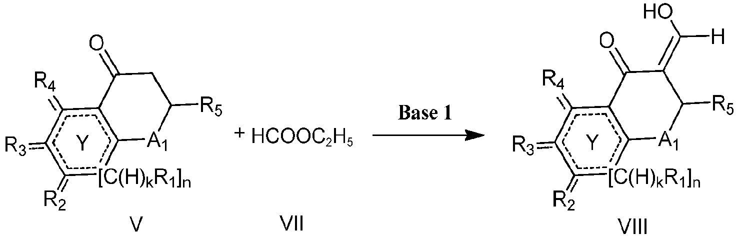 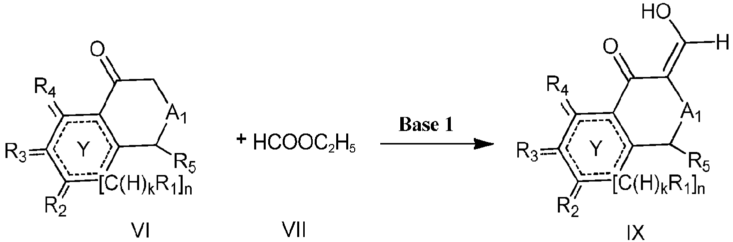
2) The cis and trans isomers of compounds XIa and XIb are prepareded by the reaction of compounds VIII with compound X'; the cis and trans isomers of compounds XIIa and XIIb are prepareded by the reaction of compounds IX with compound X'. 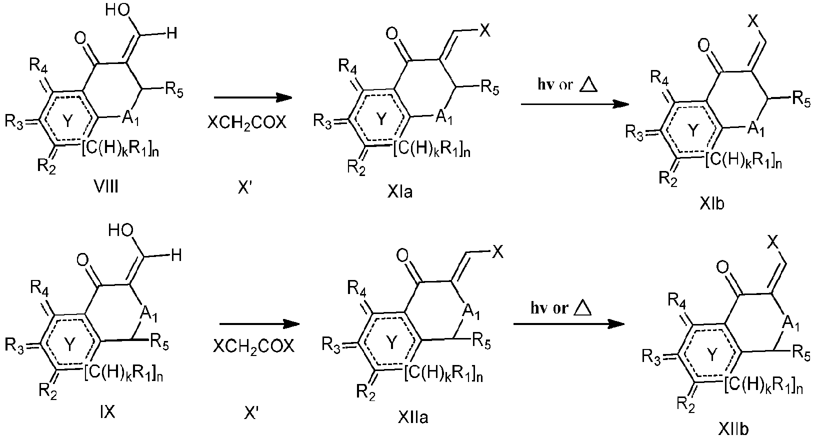
3) Compounds XIVa and XIVb were prepared by the reaction of compound XI with compound XIII and base 2. And compounds XVa and XVb were prepared by the reaction of compound XII with compound XIII and base 2. 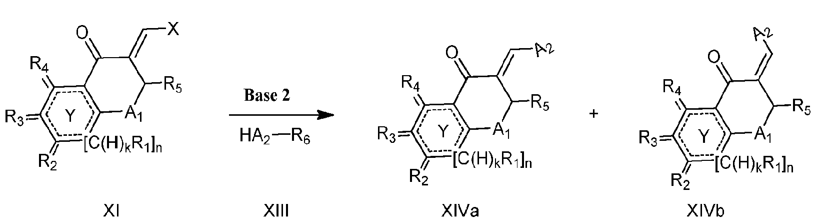 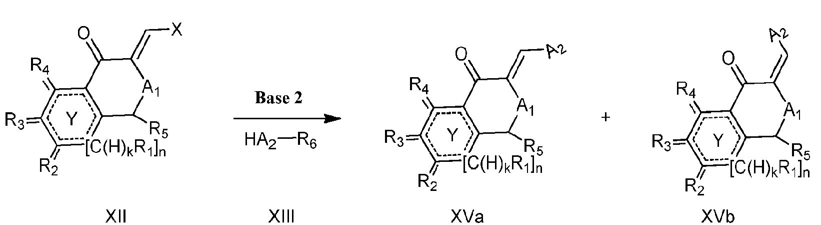
4) Compound XVII was prepared by the reaction of compound XIV with compound XVI and catalyst 1. And compound XVIII was prepared by the reaction of compound XV with compound XVI and catalyst 1. 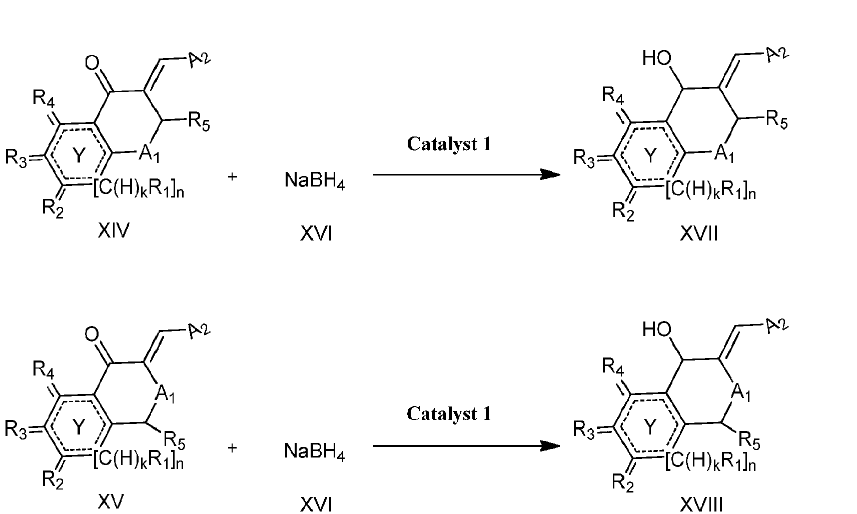
5) Compound XIX was prepared by the reaction of compound XVII with halogenation reagent. And compound XX was prepared by the reaction of compound XVIII with halogenation reagent. 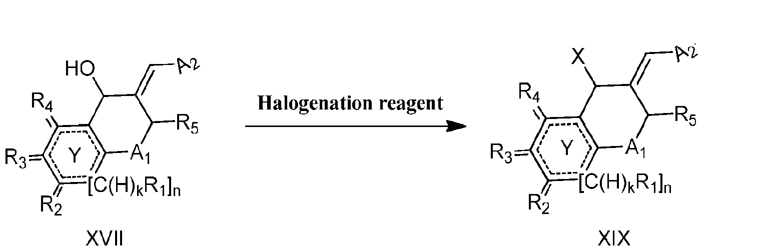 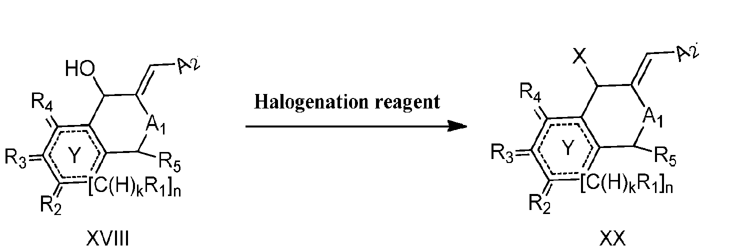
6) Compound XXII was prepared by the reaction of compound XIX with compound XXI and base 2. And compound XXIII was prepared by the reaction of compound XX with compound XXI and base 2.

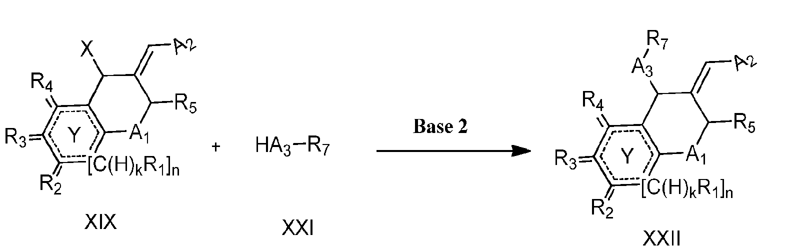

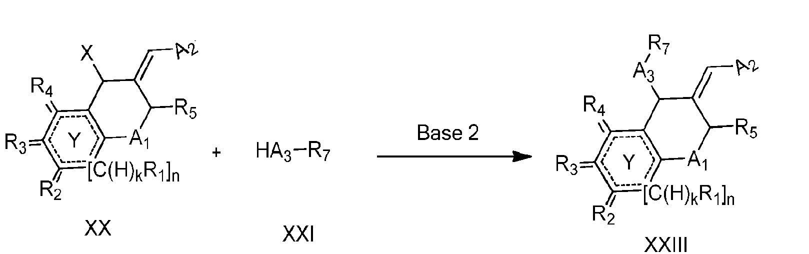

The trans-isomers of compounds XVII, XVIII, XIX, XX, XXII and XXIII could be prepared by the same methods as that the trans-isomers of compounds XIV, XV, XVII, XVIII, XIX, XX were prepared from their cis-isomer.

The definition of Y, A₁, A₂, R₁, R₂, R₃, R₄, R₅, R₇ are the same as above.

According to the content of the invention, when compounds I, II, III and IV were prepared, the mentioned base 1 can be sodium methylate, sodium ethylate, sodium hydride, and LDA, sodium methylate was preferred; the base 2 is sodium hydride; the catalyst 1 is cerium chloride; halogenation reagent could be thionyl chloride, phosphorus oxychloride or phosphorous pentachloride, and thionyl chloride is better.

According to the content of the invention, when compound I and III were prepared, the compound V and VI were added dropwise to the solution of compound VII at -78°C-0°C. And the solution can be the toluene solution of base 1 and compound VII.

According to content of the invention, when compound I and III were prepared, compounds XIa and XIIa as cis-isomer were pepared by the reaction of compound VIII and IX with compound X in sealed tube at 50 °C-80°C. The cis-isomer of compound XIa and XIIa can be transformed into compound XIb and XIIb as trans-isomer by illumination or heating, and pure compounds XIb and XIIb can be obtained by further purification.

According to the content of the invention, when compound I and III were prepared, the compound XI and XII were added dropwise to the solution of compound XIII. And the solution can be the tetrahydrofuran solution of base 2.

The usual separation methods adopted to purify the products mainly include column chromatograph, fractional crystallization, enantioseparation by chiral acid or base; moreover the pharmaceutically acceptable salt or solvate of the product can be prepared by the reaction of products with acid, base or solvent.

According to the content of the invention, when compound II and IV were prepared, the step 4 reaction was completed in ice-water bath or even lower temperature.

According to the content of the invention, when compound II and IV were prepared, the compounds XIX and XX were added dropwise to the solution of compound XXI. And the solution of compound XXI can be the tetrahydrofuran solution of base 2. The usual separation methods can be adopted to purify compounds II and IV include column chromatograph, fractional crystallization, enantioseparation by chiral acid or base. Moreover the pharmaceutically acceptable salt or solvate of the products can be prepared by the reaction of the products with acid, base or solvent.

For the technical persons in this field, the preparation of pharmaceutically acceptable salts is easy. The salts can be acid salt of the product, and the acid can be inorganic acid or organic acid. And the inorganic acid can be hydrochloric acid, hydrobromic acid, sulphuric acid, nitric acid or orthophosphoric acid; the organic acid can be succinic acid, maleic acid, acetic acid, fumaric acid, citric acid, tartaric acid, benzoic acid, p-toluenesulfonic acid, methanesulfonic acid or naphthalene sulfonic acid. In the content of the invention, all the pharmaceutically acceptable salts mentioned above include the salts of all possible stoichiometry format and unstoichiometry format.

In the content of the invention, besides pharmaceutically acceptable salts, other salts of the product can also be prepared. These salts can be used to purify other compounds, and they can be used to prepare salts of other compounds, furthermore they can be used to identifiy other compounds or as intermediate to prepare other compounds.

In the content of the invention, compounds I, II, III and IV can be crystal or amorphous. If they are crystal, they can be any kind of solvate, for example, the solvate of water. The solvate (for example, the solvate of water) may be stoichiometry, or the solvent (for example, water) contained in the compounds may be variable.

According to the content of the invention, when compound I was prepared, compound V was added dropwise to the toluene solution of compound VII at -78°C -0°C, then the mixed solution was allowed to react for 5 h. The reaction solution was extracted with aqueous solution of 5% NaOH for two times. The water layer is separated, and the pH of the water phase was adjusted to lower than 7 with hydrochloric acid. The solid of compound VIII precipitated from the solution and was obtained by filtration. Compound VIII and compound X' were dissolved in dichloromethane, and the solution was allowed to react in sealed tube at 50°C for 3 h. The reaction mixture was washed with aqueous solution of sodium carbonate for three times. The organic solvent was removed under reduced pressure, and the crude product of compound XI was obtained. The crude product was purified with silica gel column to give compound XI. Compound XIII and base 2 were dissolved in dried and pure tetrahydrofuran, and the solution was cooled with ice-water. Then compound XI was added dropwise to reaction solvent. After the reaction solvent was allowed to react for 8h, the solution was removed. The remainder was washed with water and extracted with dichlormethane. The organic solvent was removed to give the solid, and then the solid was purified with silica gel column to give compound XIV as representatives of compound I.

According to the content of the invention, when compound II were prepared, compound XIV and catalyst 1 (for example, cerium chloride) were dissolved in ethanol, then compound XVI was added at 0 °C and the reaction mixture was allowed to react for 0.5h to give compound XVII. Compound XVII and excess halogenation reagent (for example, thionyl chloride) was allowed to reflux for 2h, and then halogenation reagent was removed. The remainder was washed with water, and ectracted with organic solvent (for example, dichlormethane). Then the organic solvent was removed to give compound XIX. Compound XXI and base 2 were dissolved in dried and pure tetrahydrofuran, and the solution was cooled with ice-water. Then compound XIX was added dropwise to reaction solvent. After the reaction solvent was allowed to react for 8 h, the solvent was removed. The remainder was washed with water and extracted with dichlormethane. The organic solvent was removed to give the crude product of compound XXII, and then the crude product was purified with silica gel column to give compound XXII as representatives of compound II.

According to the content of the invention, when compound III were prepared, compound VI was added dropwise to the toluene solution of compound VII at -78 °C -0 °C, then the mixed solution was allowed to react for 5h. The reaction solution was extracted with aqueous solution of 5% NaOH for two times. The water layer is separated, and the pH of the water phase was adjusted to lower than 7 with hydrochloric acid. The solid of compound IX precipitated from the solution and was obtained by filtration. Compound IX and compound X' were dissolved in dichloromethane, and the solution was allowed to react in sealed tube at 50°C for 3 h. The reaction mixture was washed with aqueous solution of sodium carbonate for three times. The organic solvent was removed under reduced pressure, and the crude product of compound XII was obtained. The crude product was purified with silica gel column to give compound XII. Compound XIII and base 2 were dissolved in dried and pure tetrahydrofuran, and the solution was cooled with ice-water. Then compound XII was added dropwise to reaction solvent. After the reaction solution was allowed to reacted for 8h, The solvent of the reaction solution was removed. The remainder was washed with water and extracted with dichlormethane. The organic solvent in vacuo was removed to give the solid, and then the solid was purified with silica gel column to give compound XV as representatives of compound II.

According to the content of the invention, when compound IV were prepared, compound XV and catalyst 1 (for example, cerium chloride) were dissolved in ethanol, then compound XVI was added at 0 °C. And the reaction mixture was allowed to react for 0.5h to give compound XVIII. Compound XVIII and excess halogenation reagent (for example, thionyl chloride) was allowed to reflux for 2h, and then halogenation reagent was removed. The remainder was washed with water, and extracted with organic solvent (for example, dichlormethane). Then the organic solvent was removed to give compound XX. Compound XXI and base 2 were dissolved in dried and pure tetrahydrofuran, and the solution was cooled with ice-water. Then compound XX was added dropwise to reaction solvent. After the reaction solvent was allowed to react for 8 h, the solvent was removed. The remainder was washed with water and extracted with dichlormethane. The organic solvent was removed to give the crude product of compound XXIII, and then the crude product was purified with silica gel column to give compound X.

Any kind of medicine combination can be offered in this invention. The medicine combination can contain compounds of formula I, II, III and IV as new antibacterial and anticancer compounds; moreover the medicine combination can contain their derivatives, stereisomer and racemic or inracemic mixture; further the medicine combination can contain salt and solvate of these compounds, medicine adjuvant and medicine carrier chosen randomly pharmaceutically acceptable. The medicine combination can be made into dosage form to treat or prevent infection caused by fungi, to inhibit the growth of cancerous tumour cells and associated diseases in a mammal.

In the medicine combination of this invention, the effective quantity of compound I, II, III and IV can respectively be contained in suitable dosage. The medicine combination can be used to treat and prevent infection caused by fungi, to inhibit the growth of cancerous tumour cells and associated diseases in a mammal.

The effective quantity is the dosage which can hold the action of the medicine combination to treat and preventing prevent infection caused by bacteriaungi, cancer, programmed cell death, programmed cell death obstruction and tissue death induced by other factors. Generally in the medicine combinations, the good weight ratio of the compound I, II, III or IV is 0.01-80%; the better ratio is 0.05-10%; and best ratio is 0.1-5%, for example, 1-2%.

In the medicine combination of this invention, any suitable pharmaceutical adjuvant and carrier can be chosen. The pharmaceutical adjuvants and carriers can be chosen one or a mixture from oleaginous base, water-soluble base, gel ground substance, preservative, antioxidant and distilled water. Furthermore, the adjuvants and carriers include emulsifier, flavorings, pigment, propellant and others suitable for different dosage form. Moreover if humectants are need, such as glycerin, methyl glucoside and propylene glycol, glycerin and propylene glycol are preferred.

The bioactive medicine combination in this invention can be any suitable dosage form. The dosage forms include external application dosage forms and internal application dosage formss, and these dosage forms are common in this field. The external application dosage forms can be praeparatum form ointments, creams, gels, creams, lotions, suppositories or oil or spray; and the internal application dosage forms can be tablets, capsules, injections, sustained release, speed controlled release formulations or orientation controlled-release dosage forms.

The bioactive medicine combination in this invention can be prepared by a variety of methods. The methods are well known by technical people in this field, and the methods are educated in lots of technique documents, for example Remington's pharmaceutical guide can be consulted. Moreover the methods include conventional preparation techniques, such as mixing, dissolving, emulsifying and suspending agents, *et al.*

The bioactive medicine combination in this invention can be applied to a variety of animals, especially humans. For the people or animals who use the bioactive medicine combination in this invention, the dosage can be given by practitioners according to the conditions of objects, such as patients' disease level, general health, weight and age, *et al.* The bioactive medicine combination in this invention can be applied in many ways, such as through the skin, transdermal and topical application. The bioactive medicine combination in this invention can be made into fiat unguentum or gel and cream, *et al,* to treat bacteria infections through painting the medicine combination on the skin surface; the anticancer medicine combination can be made into pills, tablets, capsules, *et al,* to treat cancer cells through oral. The application frequency of the bioactive medicine combination can be affected by many factors, such as specific diseases and general health status, *et al.* Generally, 1-3 times a day are suitable for humans.

Otherwise, any kind of medicine combination was offered to treat or prevent infection caused by bacteria, to inhibit the growth of cancerous tumour cels and associated diseases in a mammal. The bioactive medicine combination can contain compounds I, II, III and IV as new antibacterial and anticancer; moreover the medicine combination can contain their derivatives, stereisomer and racemic or inracemic isomer mixture, furthermore the bioactive medicine combination can contain salt and solvate of these compound, medicine adjuvant and medicine carrier chosen randomly pharmaceutically acceptable. The bioactive medicine combination can be used on part of or all over the body.

According to the content of the invention, the bacteria mentioned above can be Blastomyces albicans, Candida tropicalis, Brewer's yeast, Microsporum gypseum, Trichoderma, Aspergillus niger, A. glaucus, Penicillium commune, Fonsecaea-Pedrosoi misdiagnosed, Cladosporium carrionii, Phialophora compacta, Phialophora verrucosa, Sporothrix schenckii, Staphylococcus aureus, Escherichia coli, gibberellin, Setosphae-ria turcica or Fusarium Oxysporum f. spvasinfectum and so on.

According to the content of the invention, the cancer cells mentioned can be gastric carcinoma, cancer of bowels, liver cancer, pancreatic cancer, esophageal carcinoma, chondrosarcoma, melanoma, Hodgkin disease, leukemia, breast carcinoma, carcinoma of prostate, carcinoma of thyroid, skin cancer or carcinoma of bladder and so on.

### DETAILED DESCRIPTION

Some examples of preparing and using the compounds in the invention were shown as the following. They can further interpret the invention. But the invention is not limited to the range of these examples.

### Examples on the general preparation method of Compounds VIII- XXII

### Example 1: the general preparation of compound VIII

Ethyl formate (36.6 mmoL) and sodium methanolate (54.9 mmoL) were dissolved in toluene, and the solution was kept at 0 °C. The toluene solution of 6-fluorothiochromanone (18.3 mmoL) was added dropwise to the reaction solution with stirring, and the mixed solution was reacted for 5h. The reaction solution was washed with 5% NaOH solution of for two times. The water layer is separated and washed with diethyl etherthen the pH of the water phase was adjusted to lower than 7. 6-fluorine-3-(hydroxylmethene)thiochroman-4-one (compound VIII; yield: 51-92%) precipitated from the solution and was obtained by filtration. Physical and chemical properties of 6-fluorine-3-(hydroxylmethene)thiochroman-4-one were listed in table 1(compound 3), and the compound is a representative of compound XVII. Compound IX can also be prepared by the method mentioned above.

### Example 2: the preparation of compound XI

6-fluoro-3-(hydroxymethylene)thiochroman-4-one(30.1 mmol) and 2-chloroacetyl chloride (45.5 mmol) were dissolved in dichloromethane, and the mixed solvent was allowed to react in the sealed tube at 50°C for 3 h. The reaction mixture was washed with aqueous solution of sodium carbonate for three times. The organic solvent was removed under reduced pressure, and the crude product of (*Z*)-3-(chloromethylene)-6-fluorothiochroman-4-one was obtained. The crude product was purified with silica gel column to give pure (Z)-3-(chloromethylene)-6- fluorothiochroman-4-one (compound 7, yield: 65-90%), and compound 7 is a representative of compound XIa. Physical and chemical properties of compound 7 were listed in table 1. And compound XIIa were prepared by the same method.

(*Z*)-3-(chloromethylene)-6-fluorothiochroman-4-one was dissolved in methanol (20 ml) in round flask. Then the solvent was illuminated with UV light for 24h. And the reaction solution were purified by silica gel column to give the (*E*)-3-(chloromethylene)-6-fluorothiochroman-4-one (compound 22, yield: 23-30%), compound 22 is a representative of compound XIb. Physical and chemical properties of compound 22 were listed in table 1. Compound XIIb were synthesized by the same method.

Their physical and chemical properties were listed in table 1. Compound XV was synthesized as the method mentioned above.

### Example 4: the preparation of compound XVII

(Z)-3-(chloromethylene)-6-methylthiochroman-4-one (22.5 mmol) and cerium chloride were dissolved in ethanol, then sodium borohydride (23.1 mmol) was added at 0°C, and the reaction mixture was allowed to react for 0.5h. Further water was added to the reaction mixture, and the mixed solvent was extracted with diethyl ether. The organic layer was removed to give (Z)-3-(chloromethylene)-6-methyl thiochroman-4-ol (compound 17; yield: 60-80 %), Physical and chemical properties were listed in table 1. (E)-3-(chloromethylene)-6-methylthiochroman-4-ol (compound 25; yield: 58-79 %) was synthesized from (E)-3-(chloromethylene)-6-methylthio- chroman-4-one by the same method as above, and Physical and chemical properties were listed in table 1, and it is one of compound XVII. Compound XVIII was synthesized as the methods mentioned above.

### Example 5: the preparation of compound XIX

(z)-3-chlormethene-6-methylthiochroman-4-ol (20.4 mmoL) in excess thionyl chloride was allowed to reflux for 2h, and then thionyl chloride was removed. The remainder was washed with water to give the products. The products were extracted with organic solvent (such as dichlormethane, *et al*), then the organic layer was removed to give (z)-4-chloro-3-(chloromethylene)-6-methylthiochroman (compound 28; yield: 53-80 %). Physical and chemical properties were listed in table 1, and it is one of compound XIX. Compound XX was synthesized as the methods mentioned above.

### Example 6: the preparation of compound XXII

### Examples on the preparation of representative compounds

6-fluoro-3-(hydroxymethylene)thiochroman-4-one (30.1mmol)) and 2-chloroacetyl chloride (45.5mmol) were dissolved in dichloromethane, and the mixed solvent was allowed to react in sealed tube at 50 °C for 3 h. The reaction mixture was washed with aqueous solution of sodium carbonate for three times. The organic solvent was removed under reduced pressure, and the crude product of (Z)-3-(chloromethylene)-6-fluorothiochroman-4-one was obtained. The crude product was purified with silica gel column to give (Z)-3-(chloromethylene)-6- fluorothiochroman-4-one (yield: 65-90%). Physical and chemical properties of it were listed in table 1 (compound 7). And compound 4-11 were prepared as the above.

(Z)-3-(chloromethylene)-6-fluorothlochroman-4-one was dissolved in methanol (30ml) in round flask. Then the solvent was illuminated with UV light for 24h. And the products were purified by silica gel column to give the (*E*)-3-(chloromethylene)-6-fluorothiochroman-4-one (yield: 23-30%). Physical and chemical properties were listed in table 1 (compound 22). Compound 21 were synthesized as the method mentioned above.

### Example 2: 3-(chloromethylene)isothiochroman-4-one

3-(hydroxymethylene)isothiochroman-4-one (30 mmol) and 2-chloroacetyl chloride (45.5mmol) were dissolved in dichloromethane, and the mixed solvent was allowed to react in sealed tube at 25°C for 3 h. The reaction mixture was washed with aqueous solution of sodium carbonate for three times. The organic solvent was removed under reduced pressure, and the crude product of (*Z*)-3-(chloromethylene)-isothiochroman-4-one was obtained. The crude product was purified with silica gel column to give (Z)-3-(chloromethylene)isothiochroman-4-one (yield: 69-90%), Physical and chemical properties of it were listed in table 1 (compound 14). And compound 15 and 16 were prepared as the method mentioned above.

(Z)-3-(chloromethylene)isothiochroman-4-one was dissolved in methanol (15ml) in round flask (50ml). Then the solvent was illuminated with UV light for 24h. And the products were purified by silica gel column to give the (*E*)-3-(chloromethylene)isothiochroman-4-one (compound 26, yield: 24-31%). Their physical and chemical properties were listed in table 1.

### Example 3: 5-(chloromethylene)-5,6-dihydro-4H-thieno[2,3-b]thiopyran-4-one

Ethyl formate (36.6 mmoL) and sodium methanolate (78.2 mmoL) were dissolved in diethyl ether, and the solution was kept at -20 °C. The diethyl ether solution of 5,6-dihydro-4H-thieno[2,3-b]thiopyran-4-one (18.3 mmoL) was added dropwise to the reaction solution with stirring, and the mixed solution was allowed to react for 12 h. The reaction solution was washed with aqueous solution of 5% NaOH for two times. The water layer is separated and washed with diethyl ether for one time. Then the pH of the water phase was adjusted to 1-2. Yellow solid (yield: 52-91%) precipitated from the solution and was obtained by filtration.

The prepared yellow solid (30.1 mmol) and 2-chloroacetyl chloride (45.5mmol) were dissolved in dichloromethane, and the mixed solvent was allowed to react in sealed tube at 50 °C for 3 h. The reaction mixture was washed with aqueous solution of sodium carbonate for three times. The organic solvent was removed under reduced pressure, and the crude product of (*Z*)- 5-(chloromethylene)-5,6-dihydro-4H-thieno[2,3-b]thiopyran-4-one was obtained. The crude product was purified with silica gel column to give (Z)-5-(chloromethylene)-5,6-dihydro-4H-thieno[2,3-b]thiopyran-4-one (yield: 53-76%). Physical and chemical properties of it were listed in table 1 (compound 19).

(*Z*)-5-(chloromethylene)-5,6-dihydro-4H-thieno[2,3-b]thiopyran-4-one was dissolved in methanol (10 ml) in round flask (50 ml). Then the solvent was illuminated with UV light for 24h. And the products were purified by silica gel column to give the (*E*)-5-(chloromethylene)-5,6-dihydro-4H-thieno[2,3-b]thiopyran- 4-one (yield: 20-32%). Itsphysical and chemical properties were listed in table 1 (compound 24).

### Example 4: 3-(chloromethylene)isothiochroman-4-ol

(Z)-3-(chloromethylene)-6-methylthiochroman-4-one (11.2 mmol) and cerium chloride were dissolved in ethanol, then sodium borohydride (11.6 mmol) was added to the reaction solution at 0°C, and the reaction mixture was allowed to react for 0.5h. Further water was added to the reaction mixture, and the mixed solvent was extracted with diethyl ether. The organic solution was removed to give (Z)-3-(chloromethylene)-isothiochroman-4-ol (yield: 76-95 %), Physical and chemical properties were listed in table 1 (compound 20). Compound 17and 19 was synthesized as the methods mentioned above, and Physical and chemical properties were listed in table 1

### Example 5: 3-(chloromethylene)-2H-thiopyrano[2,3-b]- pyridin-4(3H)-one

Ethyl formate (36.6 mmoL) and sodium hydride (78.2 mmoL) were dissolved in toluene, and the solution was kept at -20 °C. The toluene solution of 2H-thiopyrano[2,3-b]-pyridin-4(3H)-one (18.3 mmoL) was added dropwise to the reaction solution with stirring, and the mixed solution was allowed to react for 10 h. The reaction solution was washed with water for two times and with aqueous solution of 5% NaOH for one time. The water layer is separated and washed with diethyl ether for one time. Then the pH of the water phase was adjusted to lower than 7. Yellow solid yield: 51-91 %) precipitated from the solution and was obtained by filtration.

The prepared yellow solid (5.8 g) and 2-chloroacetyl chloride (45.5 mmol) were dissolved in dichloromethane, and the mixed solvent was allowed to react in sealed tube at 50°C for 3 h. The reaction mixture was washed with aqueous solution of sodium carbonate for three times. The organic solvent was removed under reduced pressure, and the crude product of (Z)-3-(chloromethylene)-2H-thiopyrano[2,3-b]- pyridin-4(3H)-one was obtained. The crude product was purified with silica gel column to give (Z)-3-(chloromethylene)-2H-thiopyrano[2,3-b]- pyridin-4(3H)-one (yield: 63-86%), It is one of compound XIa. Its physical and chemical properties were listed in table 1 (compound 18).

(*Z*)-3-(chloromethylene)-2H-thiopyrano[2,3-b]-pyridin-4(3H)-one was dissolved in methanol (30ml) in round flask (50 ml). Then the solvent was illuminated with UV light for 28h. And the products were purified by silica gel column to give the (*E*)-3-(chloromethylene)-2H-thiopyrano[2,3-b]-pyridin-4(3H)-one (yield: 19-32%). Physical and chemical properties were listed in table 1 (compound 27).

### Example 6: (Z)-3-(chloromethylene)-6-methyl-4-(p-tolylthio)thiochroman

(z)-3-chlormethene-6-methylthiochroman-4-ol (20.4 mmoL) in excess thionyl chloride was allowed to reflux for 2h, then thionyl chloride was removed. The remainder was washed with water to give the products. The products were extracted with organic solvent (such as dichlormethane, *et al*), then the organic layer was removed to give (z)-4-chloro-3-(chloromethylene)-6-methylthiochroman (compound 28, yield: 53-80%). Physical and chemical properties of compound 28 were listed in table 1.

4-methylbenzenethiol (19.1 mmoL) and sodium hydride (18.8 mmoL) were dissolved in dried and pure THF and cooled with ice-water. After the solution was stirred for 1h, the tetrahydrofuran solvent of (z)-4-chloro-3-(chloromethylene)- 6-methylthiochroman was added dropwised to reaction solvent. Then the reaction solvent was allowed to react for 8h. The solution was removed. The remainder was washed with water and extracted with dichlormethane. The organic solution was removed to give the solid mixture, then the solid was purified with silica gel column to obtain (Z)-3-(chloromethylene)-6-methyl-4-(p-tolylthio)thiochroman (compound 29, yield: 53-80%). Physical and chemical properties of compound 29 were listed in table 1.

**Table 1 Physical and chemical properties of compounds**

| **Compound** | **Molecular Formula** | **Structural Formula** | **¹H-NMR(CDCl₃)** | **ESI/APCI** |
|---|---|---|---|---|
| **1** | **C₁₀H₁₀OS** | 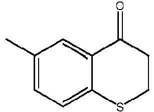 | **2.32(s,3H), 2.98(t,2H), 3.24(t,2H), 7.18(d,1H), 7.24(d,1H), 7.35(s,1H)** | **178.8** **(ESI m/z+1)** |
| **2** | **C₁₁H₉O₂S** | 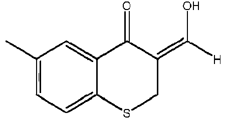 | **2.33(s,3H), 3.17(s,2H), 7.16(d,1H), 7.25(d,1H), 7.36(s,1H), 9.65(s,1H)** | **206.9** **(ESI m/z+1)** |
| **3** | **C₁₀H₆FO₂S** | 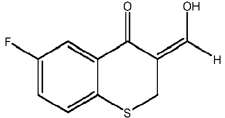 | **3.17(s,2H), 7.13-7.18(m,1H), 7.30(q,1H), 7.39(s,1H), 9.65(s,1H)** | **210.8** **(ESI m/z+1)** |
| **4** | **C₁₀H₆Cl₂OS** | 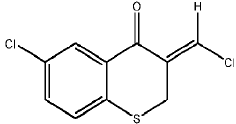 | **4.02(d,2H),7.27(d,1H), 7.38(t,2H), 8.10(d,1H)** | **244.7, 246.6** **(APCI m/z+1)** |
| **5** | **C₁₁H₉ClOS** | 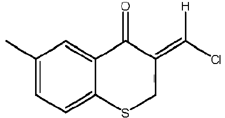 | **2.36(s,3H), 4.00(d,2H), 7.16-7.26(m,2H), 7.35(s,1H), 7.95(s,1H)** | **224.6, 226.6** **(APCI m/z+1)** |
| **6** | **C₁₁H₈ClFOS** | 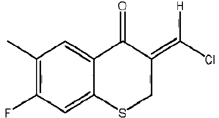 | **2.30(d,3H), 4.00(d,2H), 7.10(d,1H), 7.36(s,1H), 7.76(d,1H)** | **242.7, 244.7** **(APCI m/z+1)** |
| **7** | **C₁₀H₆ClFOS** | 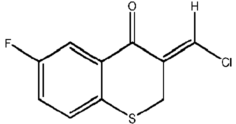 | **4.01(d,2H), 7.13-7.18(m,1H) 7.30(q,1H), 7.38(s,1H), 7.81(dd,1H)** | **228.8, 230.8** **(APCI m/z+1)** |
| **8** | **C₁₁H₉BrOS** | 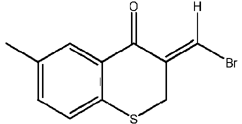 | **2.34(s,3H), 3.99(d,2H), 7.16-7.23(m,2H), 7.57(s,1H), 7.927(d,1H)** | **268.6, 270.6** **(APCI m/z+1)** |
| **9** | **C₁₁H₈BrFOS** | 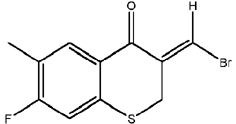 | **2.30(d,3H), 4.01(d,2H), 7.14(d,1H), 7.61(s,1H), 7.76(d,1H)** | **286.6, 288.6** **(APCI m/z+1)** |
| **10** | **C₁₀H₆BrClOS** | 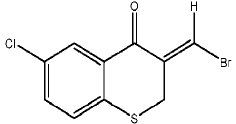 | **4.03(s,2H), 7.27(d,1H), 7.38(dd,1H), 7.64(s,1H), 8.10(d,1H)** | **288.7, 290.7** **(APCI m/z+1)** |
| **11** | **C₁₀H₆BrFOS** | 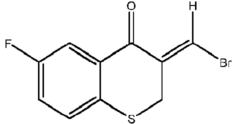 | **4.02(s,2H), 7.13-7.18(m,1H), 7.30(q,1H), 7.64(s,1H), 7.81(dd,1H)** | **272.7, 274.7** **(APCI m/z+1)** |
| **13** | **C₁₀H₁₀OS** | 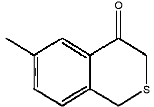 | **2.32(s,3H), 3.75(d,2H), 3.67(d,2H), 7.18(d,1H), 7.24(d,1H), 7.35(s,1H)** | **178.8** **(ESI m/z+1)** |
| **14** | **C₁₀H₇ClOS** | 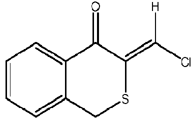 | **3.85(s,2H), 7.25(d,1H), 7.40(s,1H), 7.45(dd,1H), 7.97(d,1H), 8.7(d,1H)** | **224.6, 226.6** **(APCI m/z+1)** |
| **15** | **C₁₀H₆ClFOS** | 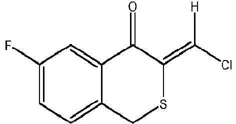 | **3.86(s,2H), 7.41(s,1H), 7.30(dd,1H), 8.01(d,1H), 8.87(s,1H)** | **228.8, 230.8** **(APCI m/z+1)** |
| **16** | **C₁₁H₈BrFOS** | 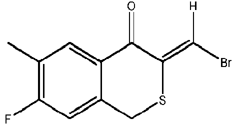 | **2.30(d,3H), 4.13(d,2H), 7.14(d,1H), 7.61(s,1H), 7.76(dd,1H)** | **286.6, 288.6** **(APCI m/z+1)** |
| **17** | **C₁₁H₁₁ClOS** | 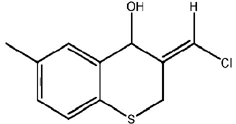 | **2.35(s,3H), 2.49(s,1H), 4.01(s,2H), 4.87(s,1H), 7.16-7.27(m,2H), 7.36(s,1H), 7.95(s,1H)** | **225.8, 227.8** **(ESI m/z+1)** |
| **18** | **C₉H₆ClNOS** | 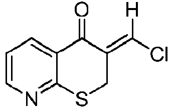 | **3.74(d,2H), 7.01(dd,1H), 7.87(s,1H), 7.96(dd,1H), 8.32(dd,1H)** | **211.7, 213.7** **(ESI m/z+1)** |
| **19** | **C₈H₅ClOS₂** | 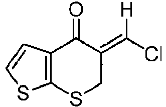 | **4.17(s,2H), 7.07-7.08(m,1H), 7.37(s,1H), 7.49-7.50(m,1H)** | **216.7, 218.7** **(ESI m/z+1)** |
| **20** | **C₁₀H₉ClOS** | 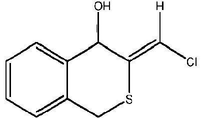 | **3.65(s,1H), 4.13(s.2H), 5.19(d,1H), 5.87(s,1H), 7.16-7.19(m,2H), 7.27-7.26(m,2H)** | **212.7, 214.7** **(APCI m/z+1)** |
| **21** | **C₁₀H₆Cl₂OS** | 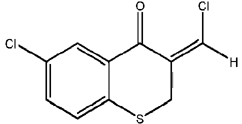 | **3.87(s,2H), 6.76(s,1H), 7.25(d,1H), 7.39(dd,1H), 8.19(d,1H)** | **228.8, 230.8** **(APCI m/z+1)** |
| **22** | **C₁₀H₆ClFOS** | 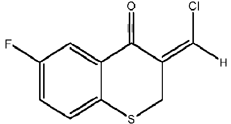 | **3.90(s,2H), 6.78(s,1H), 7.13-7.18(m,1H), 7.30(q,1H), 7.81(dd,1H)** | **228.7, 230.7** **(APCI m/z+1)** |
| **24** | **C₈H₅ClOS₂** | 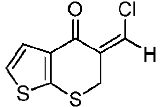 | **4.02(s,2H), 7.08(d,1H), 6.81(s,1H), 7.49(d,1H)** | **216.8, 218.8** **(APCI m/z+1)** |
| **25** | **C₁₁H₁₁ClOS** | 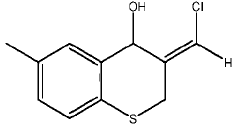 | **2.34(s,3H), 3.44(q,2H), 3.65(s,1H), 5.19(d,1H), 5.82(s,1H), 7.06(dd,1H), 7.13(d,1H), 7.20(d,1H)** | **226.7, 228.7** **(APCI m/z+1)** |
| **26** | **C₁₀H₇ClOS** | 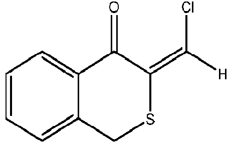 | **3.86(s,2H), 7.23(d,1H), 7.42(d,1H), 7.51(dd,1H), 7.94(d,1H), 8.73(d,1H)** | **210.8, 212.8** **(APCI m/z+1)** |
| **27** | **C₉H₆ClNOS** | 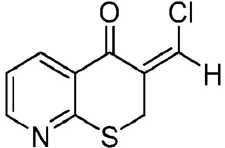 | **3.91(d,2H), 6.99(dd,1H), 7.52(s,1H), 7.95(dd,1H), 8.34(dd,1H)** | **211.7, 213.7** **(APCI m/z+1)** |
| **28** | **C₁₁H₁₀Cl₂S** | 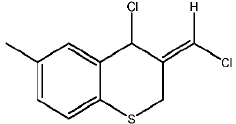 | **2.34(s,3H), 3.44(q,2H), 5.44(s,1H), 5.76(s,1H), 7.13(dd,1H), 7.20(d,1H), 7.27(d,1H)** | **244.7, 246.7** **(APCI m/z+1)** |
| **29** | **C₁₈H₁₇ClS₂** | 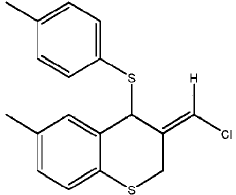 | **2.31(s,3H), 2.35(s,3H), 3.45(q,2H), 4.50(s,1H), 5.76(s,1H), 6.94-7.00(m,2H), 7.13-7.15(dd,2H), 7.26-7.28(m,2H)** | **332.8, 334.8** **(APCI m/z+1)** |

### Antibacterial activity experiment:

In vitro antibacterial experiments of 25 compounds as anti-bacterial and anti-cancer compounds in the patent were tested using 14 kinds of bacteria. And the results obtained by two-fold dilution method were shown in table 2.

Tested bacteria: C. albicas, C. tropicalis, C. neoformans, E. floccosum, M. gypseum, Aniger, S. schenekn, C. parapsilosis, C. glabrata, C. Krusei, Trichoderma, Gibberella, Setrosphaeriaturcica, Fusarium OxysporumVasinfectum. (Bacteria were from the Dermatology Hospital of Chinese Academy of Medical Sciences, Nanjing, China.)

The Preparation of tested Bacteria solution was following: incubated Bacteria were added to 5ml physiological saline, and then they were mashed and placed with ultrasound. The solution was fully mixed, and insoluble substances were removed. The treated solutions were called the original bacterium solution. In the test, the bacteria concentrations in the original bacterium solution were adjusted to 10-6cells/ml.

Tested Methods: the tested compounds were dissolved in dimethyl sulfoxide and the solution was diluted with sterile distilled water. And the distilled solution was added to sterilized RPMI 1640 medium. The concentration of the compounds were adjusted to 256, 128, 64, 32, 16, 8, 4, 2, 1, 0.5, 0.25, 0.125 ug*ml-1. Then the adjusted solutions were inoculated with tested bateria, and the tested systems were placed in the constant temperature oven to culture for 2-7 days. The concentration at which there was no growing of fungi was taken as the minimum inhibitory concentration (MIC).

In vitro anticancer experiments of 25 compounds as anti-bacterial and anti-cancer compounds in the patent were tested. In the test, 14 kinds of cancer cells were used as tested cancer cells. And the results obtained by MTT method were shown in table 2 (the inhibition ratio was obtained when the concentration was 5ug/ml).

Tested Cancer Cells: SGC7901, HTB-38HT-29, CRL-2233NU-398, CRL-1469PANC-1, B0192, HTB-131MDA-453, CRL-1435PC-3, FTC133, AS24391, HTB-95637. (Cancer cells were from Chinese Military Academy of Medical Sciences).

Cells Culture: Culture solution was composed of RPMI 1640 medium, 10 % (V/V) Fetal calf serum and 0.01% L-glutamine. Cultured cells were maintained at 37 °C in an incubator with 5% CO2. The cells at logarithmic growth phase were used in the experiment.

MTT Experiment: Single cell suspension was obtained by digesting cells with 0.25% trypsin. The cells at logarithmic growth phase were collected and seeded in a 96-well plate at a concentration of 6000-7000 cells per well. After culturing for 12 h at 37 °C in the incubator with 5% CO2, the cells were again incubated with the tested compounds of various concentrations for 48 h or 72 h. Pure cultures and cells without drug were taken as blank reference and negative reference, and 8 Wells of each group were taken. Twenty microliter of MTT (5 mg/mL) was added to each well, and the mixed solutions were incubation for 4 h. The formazan product was dissolved by dimethyl sulfoxide (DMSO, 150 µL), and the optical density (O.D.) was read at 570 nm.

The Inhibition Ratio was calculated by the following formula: $Inhibition Ratio = \left({A}_{negative control}-{A}_{sample}\right) / \left({A}_{negative control}-{A}_{blank control}\right)$

From the results it can be seen that the new series of anti-bacterial and anti-cancer drug compounds all have inhibition activity of different degrees to bateria and cancer cells.

In short, all the drugs of this invention are synthesized from thiochromanones (or substituted- thiochromanones). And all of the chemical reagents used in synthesis process were common and easy to be purchased. The pharmacology and toxicology experiments showed that the drugs in this invention had a certain inhibition activity to bateria and cancer cells

The compounds in this invention can be widely used in antimicrobial and anticancer field. And there was a broad research value and application prospects for these compounds.

The invention was described by the way of example explanation. But it should be understood that the invention is not only limited in these specific examples. The skilled person can make various modifications to the invention.

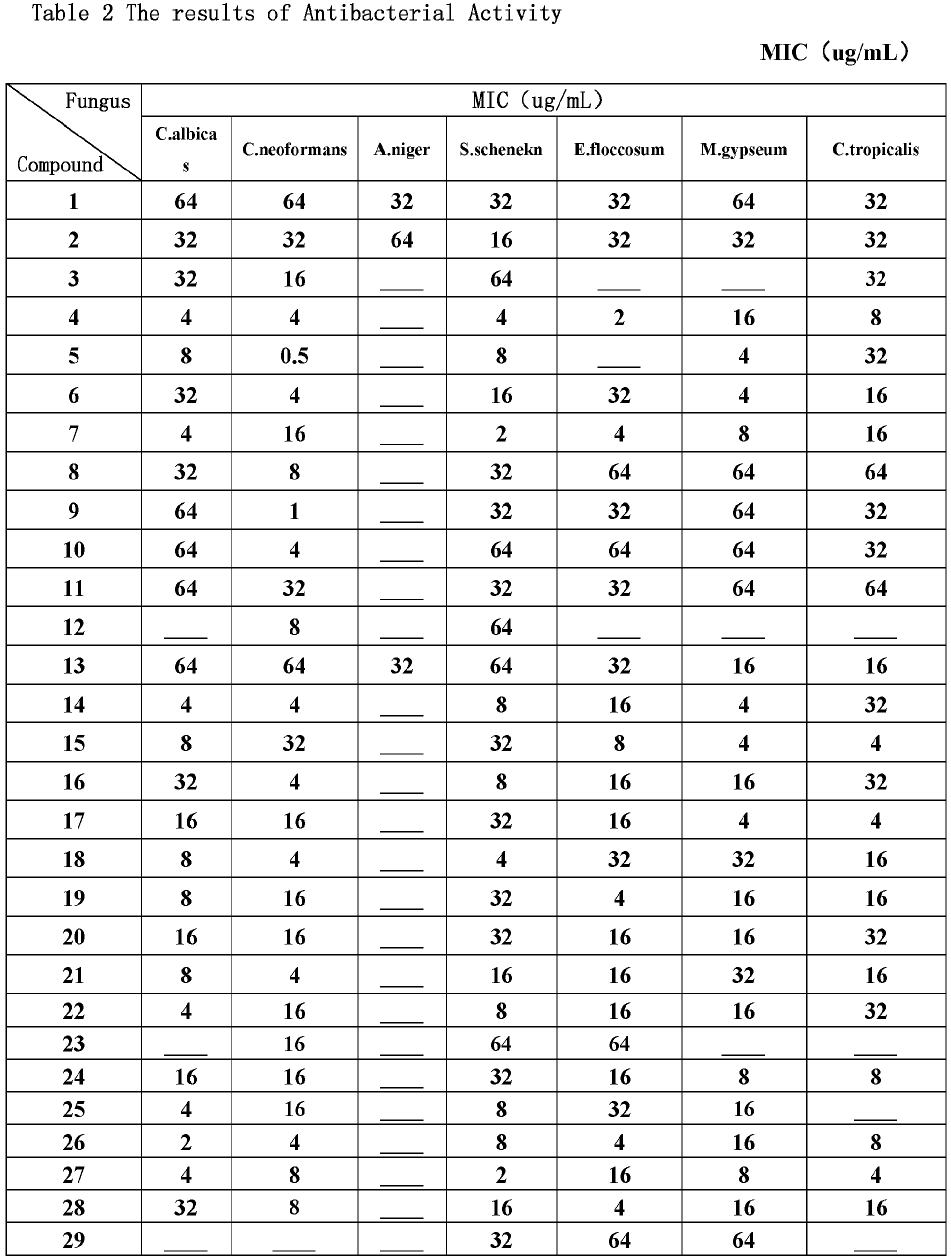

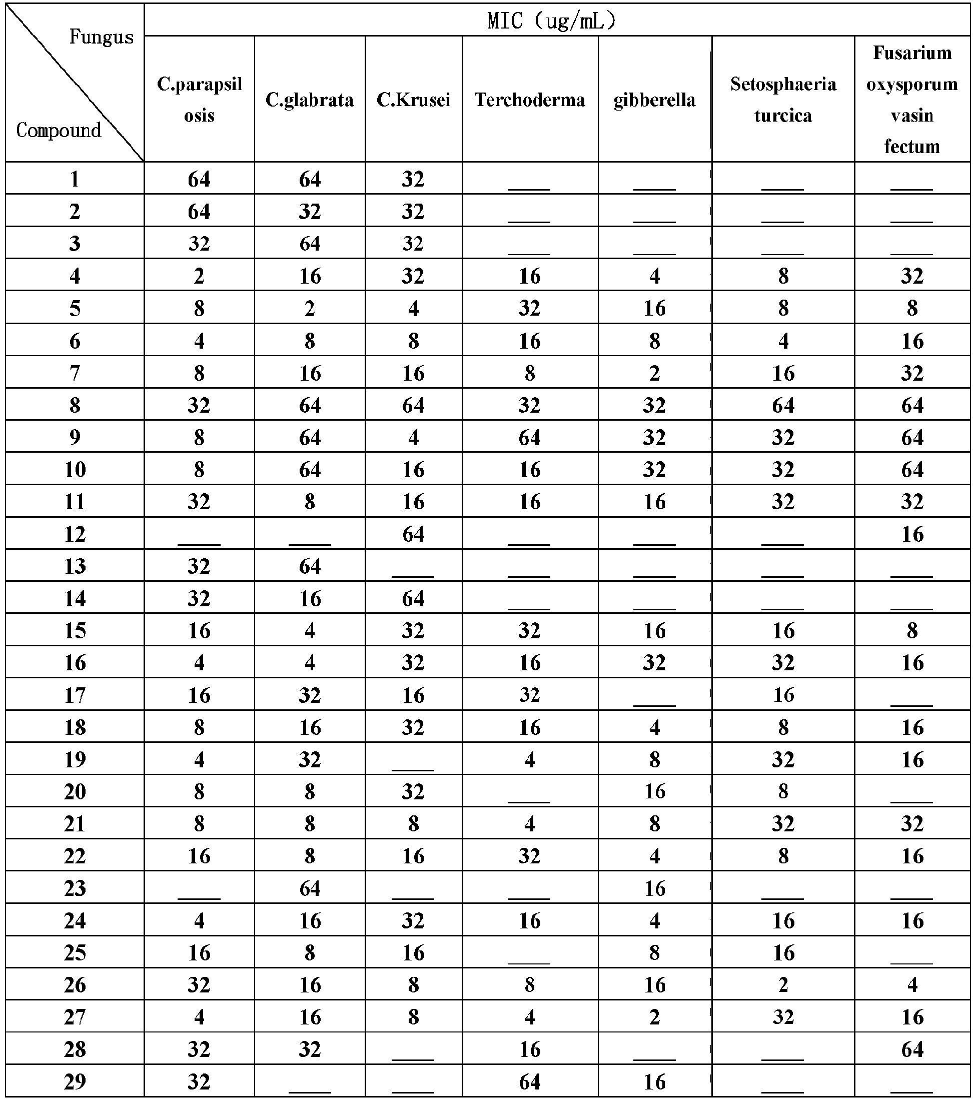

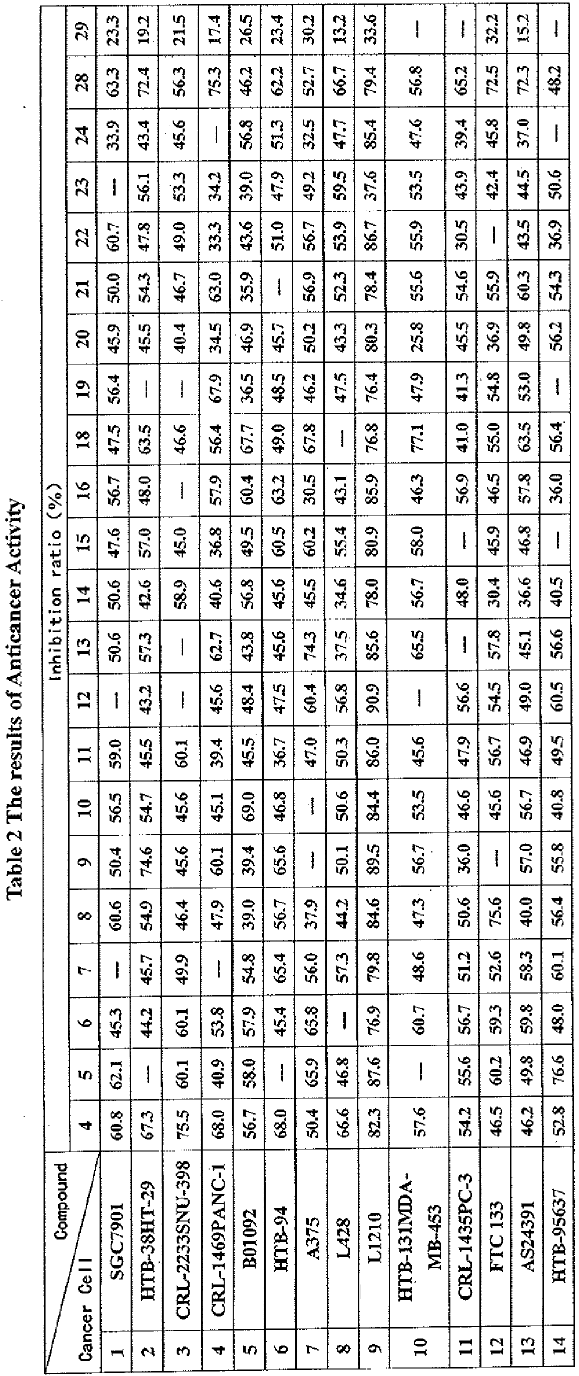

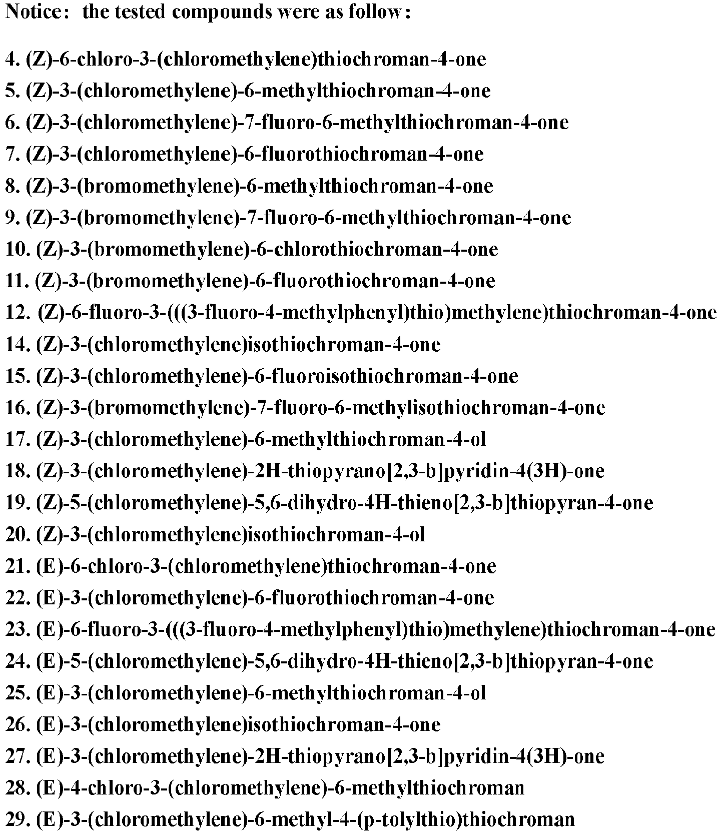

## Claims

1. A novel antibiotic and anticancer compound of general formula I, II, III or IV, derivatives, stereoisomers, the racemic or non-racemic mixture of the stereoisomers or the pharmaceutically acceptable salt or solvate of the compounds, the general formula is shown as following: 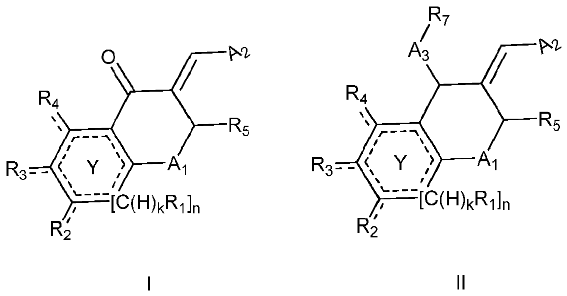 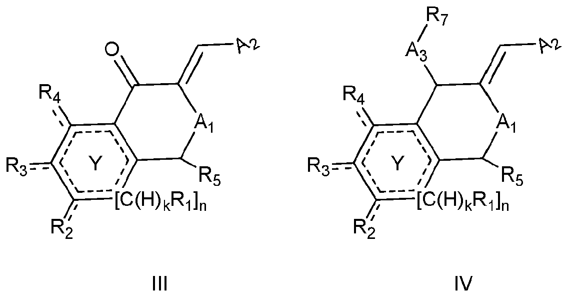
wherein Y represents the elements of benzene ring at any position, and it can be independently selected from C, O, S and N; when Y stands for O or S, it is bivalent element; Y is trivalent element when it stands for N; and it is quadrivalent element when it stands for C, Y is preferably selected from C, N or S;
the dotted lines represent the bonds which are dispensable, when a bond is double bond, its neighbor bonds are not double bonds;
k is an integer 0 or 1; n is an integer 0, 1 or 2;
R₁, R₂, R₃ and R₄ can be independently selected from hydrogen, fluorine, chlorine, bromine, iodine, hydroxyl, cyano, C₁₋₂₀-alkyl, C₁₋₂₀-alkyl-oxy, C₁₋₂₀-alkyl carbonyl, and C₁₋₂₀-alkyl-carbonyl-oxy, on condition that at least one of R₁, R₂, R₃ and R₄ is not hydrogen, and the alkyl portion in these groups can be replaced by one or more of the independent halogen atoms such as fluorine, chlorine, bromine and iodine;
R₅ represents hydrogen, C₁₋₂₀-alkyl, C₁₋₂₀-alkyl-oxy, C₁₋₂₀-alkyl-carbonyl, or C₁₋₂₀-alkyl-carbonyl-oxy;
R₇ represents hydrogen, alkyl, aryl, substituted aryl or heteroaryl;
A₁ represents CH₂, CH₂CH₂, O, S, S(O), S(O)₂, or NR₁;
A₂ represents Cl, Br, F or I;
A₃ represents O, S, S(O), S(O)₂, NR, Cl, Br, F, I or P; when A₃ is Cl, Br, F or I, R₇ does not exist.
wherein C₁₋₂₀-hydrocarbonyl is aromatic hydrocarbon group or non-aromatic hydrocarbonyl, straight chain hydrocarbonyl or branched chain hydrocarbonyl, cyclic hydrocarbonyl or non-cyclic hydrocarbonyl.

2. The novel antibiotic and anticancer compound of general formula I, II, III or IV , derivatives, stereoisomers, the racemic or non-racemic mixture of the stereoisomers, or the pharmaceutically acceptable salt or solvate of the compound of claim 1, wherein C₁₋₂₀ hydrocarbonyl is selected from C₁₋₂₀-alkyl, C₂₋₂₀-alkenyl, C₂₋₂₀-alkynyl, C₃₋₂₀-cycloalkanyl, C₃₋₂₀-cycloalkenyl, C₆₋₂₀-aryl, C₆₋₁₀-aryl-C₁₋₁₀-alkyl, C₃₋₁₀-cyclo-alkanyl-C₁₋₁₀-alkyl, C₃₋₁₀-cycloalkenyl- C₁₋₁₀-alkyl and C₁₋₁₀-alkyl-C₆₋₁₀- aryl.

3. The novel antibiotic and anticancer compound of general formula I, II, III or IV , derivatives, stereoisomers, the racemic or non-racemic mixture of the stereoisomers, or the pharmaceutically acceptable salt or solvate of the compound of claim 1 or 2, wherein compound I is the stereoisomer or mixture of compound Ia and Ib; compound II is the stereoisomer or mixture of compound IIa and IIb; compound III is the stereoisomer or mixture of compound IIIa and IIIb, compound IV is the stereoisomer or mixture of compound IVa and IVb. 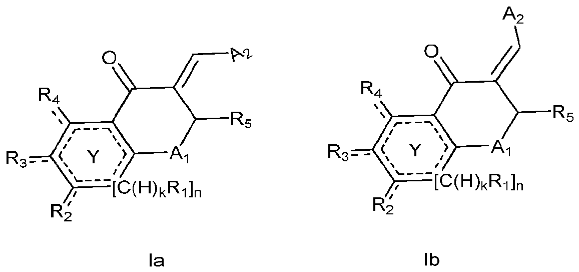 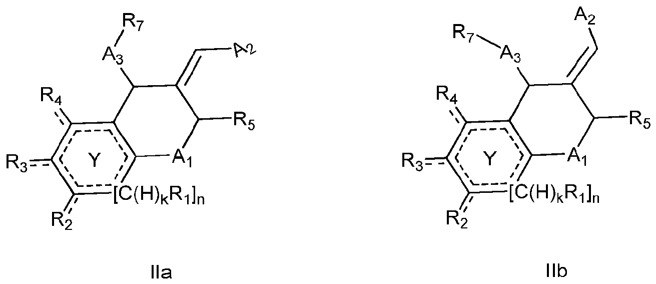 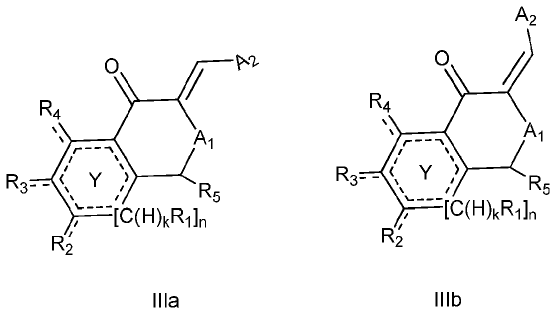 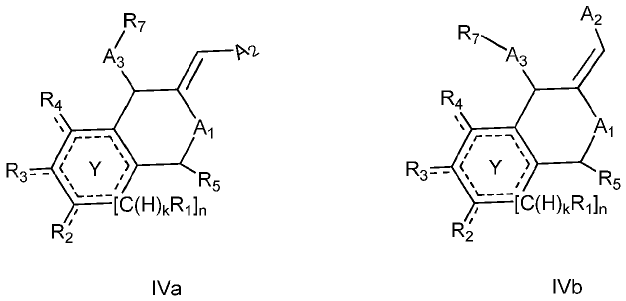 wherein, the definitions of Y, A₁, A₂, A₃, R₁, R₂, R₃, R₄, R₅, R₆, R₇, k and n are the same as that of claim 1 or 2.

4. The novel antibiotic and anticancer compound of general formula I, II, III or IV , derivatives, stereoisomers, the racemic or non-racemic mixture of the stereoisomers, or the pharmaceutically acceptable salt or solvate of the compound of claim 1, wherein new anti-bacterial, anti-cancer compounds that are represented by compounds of formula I, II, III or IV include the following:
**(Z)-6-chloro-3-(chloromethylene)thiochroman-4-one**
**(Z)-3-(chloromethylene)-6-methylthiochroman-4-one**
**(Z)-3-(chloromethylene)-7-fluoro-6-methylthiochroman-4-one**
**(Z)-3-(chloromethylene)-6-fluorothiochroman-4-one**
**(Z)-3-(bromomethylene)-6-methylthiochroman-4-one**
**(Z)-3-(bromomethylene)-7-fluoro-6-methylthiochroman-4-one**
**(Z)-3-(bromomethylene)-6-chlorothiochroman-4-one**
**(Z)-3-(bromomethylene)-6-fluorothiochroman-4-one**
**(Z)-6-fluoro-3-(((3-fluoro-4-methylphenyl)thio)methylene)thiochroman-4-one**
**(Z)-3-(chloromethylene)isothiochroman-4-one**
**(Z)-3-(chloromethylene)-6-fluoroisothiochroman-4-one**
**(Z)-3-(bromomethylene)-7-fluoro-6-methylisothiochroman-4-one**
**(Z)-3-(chloromethylene)-6-methylthiochroman-4-ol**
**(Z)-3-(chloromethylene)-2H-thiopyrano[2,3-b]pyridin-4(3H)-one**
**(Z)-5-(chloromethylene)-5,6-dihydro-4H-thieno[2,3-b]thiopyran-4-one**
**(Z)-3-(chloromethylene)isothiochroman-4-ol**
**(E)-6-chloro-3-(chloromethylene)thiochroman-4-one**
**(E)-3-(chloromethylene)-6-fluorothiochroman-4-one**
**(E)-6-fluoro-3-(((3-fluoro-4-methylphenyl)thio)methylene)thiochroman-4-one**
**(E)-5-(chloromethylene)-5,6-dihydro-4H-thieno[2,3-b]thiopyran-4-one**
**(E)-3-(chloromethylene)-6-methylthiochroman-4-ol**
**(E)-3-(chloromethylene)isothiochroman-4-one**
**(E)-3-(chloromethylene)-2H-thiopyrano[2,3-b]pyridin-4(3H)-one**
**(E)-4-chloro-3-(chloromethylene)-6-methylthiochroman**
**(E)-3-(chloromethylene)-6-methyl-4-(p-tolylthio)thiochroman**

5. A preparation method of said novel antibiotic and anticancer compound of general formula I, II, III or IV , derivatives, stereoisomers, the racemic or non-racemic mixture of the stereoisomers, or the pharmaceutically acceptable salt or solvate of the compound, comprising the following:
1) the compounds VIII is prepared by the reaction of compounds V with compound VII and Base 1; the compounds IX is prepared by the reaction of compounds VI with compound VII and Base 1. 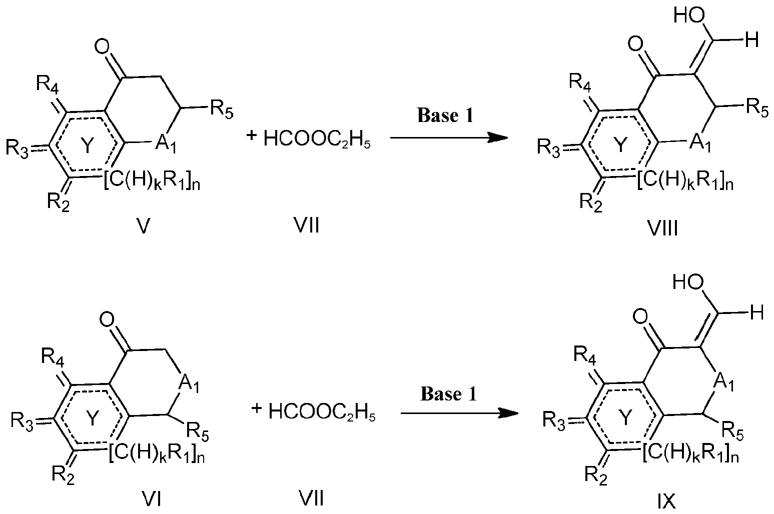
2) the cis- and trans- isomers of compounds XIa and XIb are prepared by the reaction of compounds VIII with compound X' and the cis- and trans- isomers of compounds XIIa and XIIb are prepared by the reaction of compounds IX with compound X'; 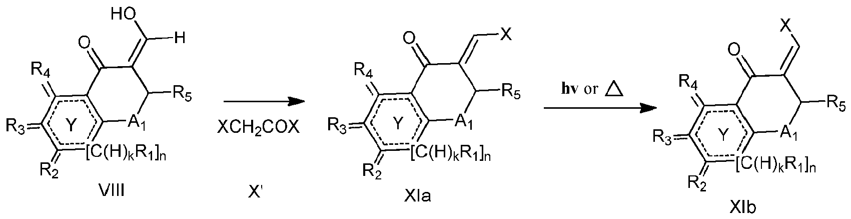 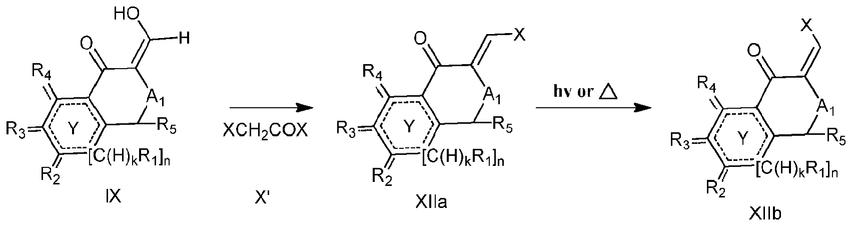
3) compound XVII are prepared by the reaction of compound XIV with compound XVI and catalyst 1, and compound XVIII are prepared by the reaction of compound XV with compound XVI and catalyst 1; 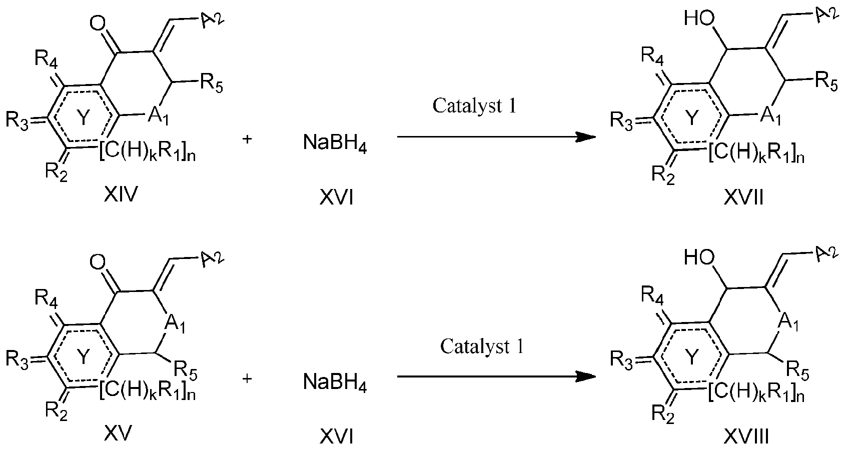
4) compound XIX is prepared by the reaction of compound XVII with halogenation reagent, and compound XX is prepared by the reaction of compound XVIII with halogenation reagent; 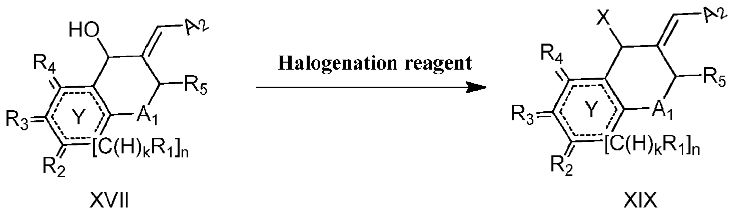 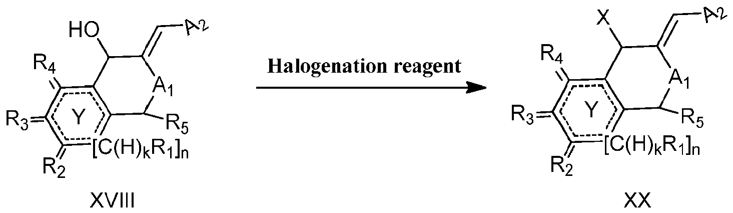
5) compound XXII is prepared by the reaction of compound XIX with compound XXI and base 2, and compound XXIII is prepared by the reaction of compound XX with compound XXI and base 2; 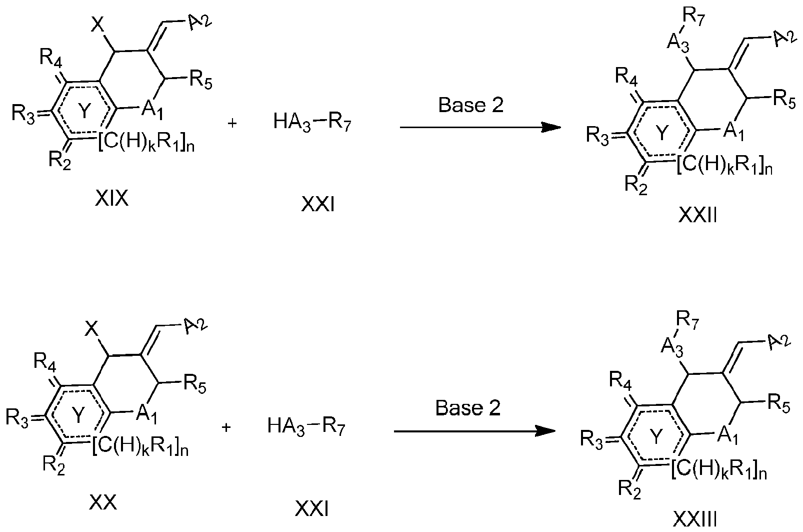
trans-isomers of compounds XIV, XV, XVII, XVIII, XIX and XX can be prepared as the methods of their cis-isomers preparation, and trans-isomers of compound XVII, VIII, IX (XX), (XXII) and XXIII can be obtained as the ways mentioned above;
wherein, Y, A₁, A₂, A₃, R₁, R₂, R₃, R₄, R₅, R₇, k, n and the definition of dotted line are the same as that of claim 1 or 2; X is halogen.

6. A medicine combination of novel antibiotic and anticancer compound of general formula I, II, III or IV , derivatives, stereoisomers, the racemic or non-racemic mixture of the stereoisomers, or the pharmaceutically acceptable salt or solvate of the compound of claim 1, 2, 3, 4 or 5, pharmaceutical adjuvants or optional pharmaceutical carrier.

7. The medicine combination of claim 6, wherein the dosage form of the medicine combination can be praeparatum form ointment, cremor, gelata, cream, lotion, uppos, oils, massa pilularum, tablets, collocystis, injectable preparation and consperge agent.

8. Compound of general formula I, II, III or IV, derivatives, stereoisomers, the racemic or non-racemic mixture of the stereoisomers, or the pharmaceutically acceptable salt or solvate of the compound of claim 1, 2, 3 or 4, for use as antibiotic and anticancer medicine in treating or preventing infection caused by bacteria, inhibiting the growth of cancerous tumour cells or associated diseases in a mammal.

9. The compounds of claim 8 for use, wherein said bacteria include blastomyces albicans, candida tropicalis, bakers' yeast, cryptococcus neoformans, acrothesium floccosum, trichophyton gypseum, trichophyton rubrum, trichophyton tonsurans, microsporum gypseum, trichoderma, aspergillus niger, A. glaucus, penicillium commune, Fonsecaea-Pedrosoi misdiagnosed, cladosporium carrionii, phialophora compacta, phialophora verrucosa, sporothrix schenckii, staphylococcus aureus, bacillus coli, erythro-mould, big block bristle cavity spot fungus and fusarium fungi, et al., said cancer cells include gastric cancer, bowels cancer, liver cancer, pancreatic cancer, esophageal cancer B01092, chondroma sarcomatosum, melanoma, Hodgkin disease, leukemia, breast cancer, prostatic carcinoma, thyroid cancer, cutaneous cancer and carcinoma of bladder, *etc.*

10. Use of said novel antibiotic and anticancer compound of general formula I, II, III or IV, derivatives, stereoisomers, the racemic or non-racemic mixture of the stereoisomers, or the pharmaceutically acceptable salt or solvate of the compound of claim 1, 2, 3 or 4, for preparing medicine which can be used to treat or prevent infection caused by bacteria, to inhibit the growth of cancerous tumour cells and associated diseases in a mammal.

## Patentansprüche

1. Neuartige antibiotische und Antikrebs-Verbindung mit der allgemeinen Formel I, II, III oder IV, Derivate, Stereoisomere, die racemische oder nicht racemische Mischung der Stereoisomere oder das pharmazeutisch akzeptable Salz oder Solvat der Verbindungen, wobei die allgemeine Formel wie folgt angegeben wird: 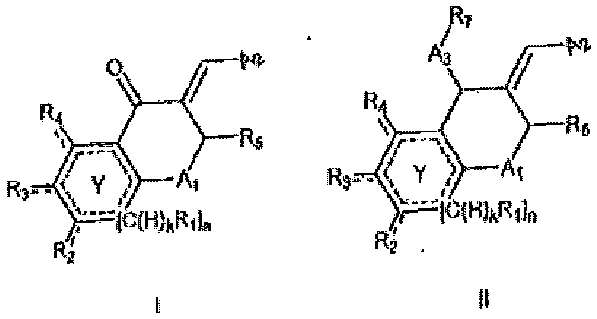 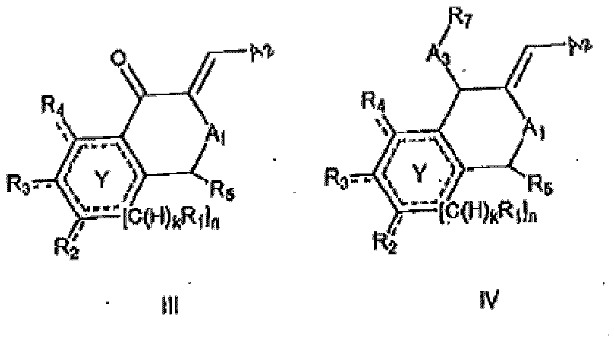
wobei Y die Elemente des Benzenrings an jeder Position darstellt und unabhängig aus C, O, S und N ausgewählt werden kann; wenn Y für O oder S steht, handelt es sich um ein bivalentes Element; Y ist ein trivalentes Element, wenn es für N steht; und es ist ein quadrivalentes Element, wenn es für C steht, Y wird vorzugsweise aus C, N oder S ausgewählt;
die gepunktete Linen stellen die Bindungen dar, die entbehrlich sind, wenn eine Bindung eine Doppelbindung ist, sind ihre benachbarten Bindungen keine Doppelbindungen;
k ist eine ganze Zahl 0 oder 1; n ist eine ganze Zahl 0, 1 oder 2;
R₁, R₂, R₃ und R₄ können unabhängig ausgewählt sein aus Wasserstoff, Fluor, Chlor, Brom, Hydroxyl, Cyan, C₁₋₂₀-Alkyl, C₁₋₂₀-Alkyloxy, C₁₋₂₀-Alkylcarbonyl und C₁₋₂₀-Alkylcarbonyloxy, unter der Bedingung, dass mindestens eines von R₁, R₂, R₃ und R₄ kein Wasserstoff ist und der Alkylteil in diesen Gruppen durch ein oder mehrere unabhängige Halogenatome ersetzt werden kann, wie z.B. Fluor, Chlor, Brom und Iod;
R₅ Wasserstoff, C₁₋₂₀-Alkyl, C₁₋₂₀-Alkyloxy, C₁₋₂₀-Alkylcarbonyl oder C₁₋₂₀-Alkylcarbonyloxy darstellt;
R₇ Wasserstoff, Alkyl, Aryl, substituiertes Aryl oder Heteroaryl darstellt;
A₁ gleich CH₂, CH₂CH₂, O, S, S(O), S(O)₂ oder NR₁ darstellt;
A₂ gleich Cl, Br, F oder I darstellt;
A₃ gleich O, S, S(O), S(O)₂, NR, Cl, Br, F, I oder P darstellt; wenn A₃ gleich Cl, Br, F oder I ist, R₇ ist nicht vorhanden,
wobei C₁₋₂₀-Hydrocarbonyl eine aromatische Kohlenwasserstoffgruppe oder ein nicht aromatisches Hydrocarbonyl ist, ein geradkettiges oder ein verzweigtes Hydrocarbonyl, ein zyklisches Hydrocarbonyl oder ein nicht zyklisches Hydrocarbonyl.

2. Neuartige antibiotische und Antikrebs-Verbindung mit der allgemeinen Formel I, II, III oder IV, Derivate, Stereoisomere, die racemische oder nicht racemische Mischung der Stereoisomere oder das pharmazeutisch akzeptable Salz oder Solvat der Verbindung nach Anspruch 1, wobei C₁₋₂₀-Hydrocarbonyl ausgewählt ist aus C₁₋₂₀-Alkyl, C₂₋₂₀-Alkenyl, C₂₋₂₀-Alkynyl, C₃₋₂₀-Cycloalkanyl, C₃₋₂₀-Cycloalkenyl, C₆₋₂₀-Aryl, C₆₋₁₀-Aryl-C₁₋₁₀-Alkyl, C₃₋₁₀-Cycloalkanyl-C₁₋₁₀-Alkyl, C₃₋₁₀-Cycloalkenyl-C₁₋₁₀-Alkyl und C₁₋₁₀-Alkyl-C₆₋₁₀-Aryl.

3. Neuartige antibiotische und Antikrebs-Verbindung mit der allgemeinen Formel I, II, III oder IV, Derivate, Stereoisomere, die racemische oder nicht racemische Mischung der Stereoisomere oder das pharmazeutisch akzeptable Salz oder Solvat der Verbindungen nach Anspruch 1 oder 2, wobei die Verbindung I das Stereoisomer oder die Mischung von Verbindung Ia und Ib ist; die Verbindung II das Stereoisomer oder die Mischung von Verbindung IIa und IIb ist; die Verbindung III das Stereoisomer oder die Mischung von Verbindung IIIa und IIIb ist, die Verbindung IV das Stereoisomer oder die Mischung von Verbindung IVa und IVb ist, 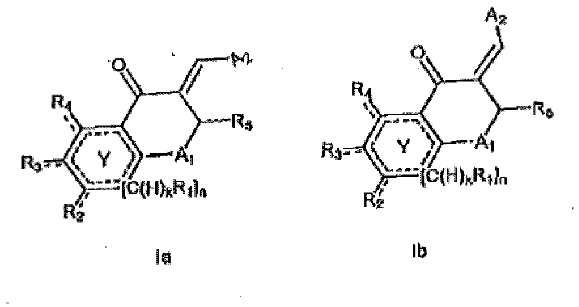 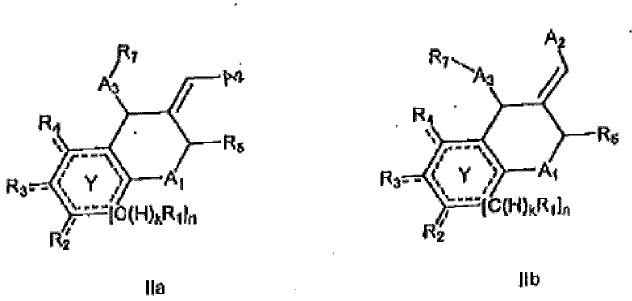 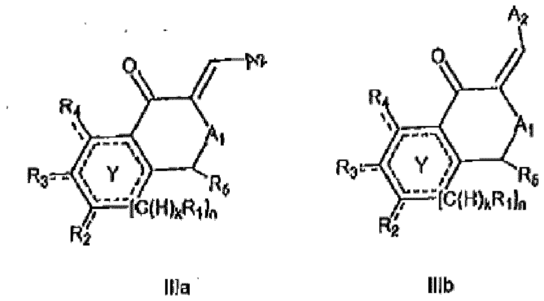 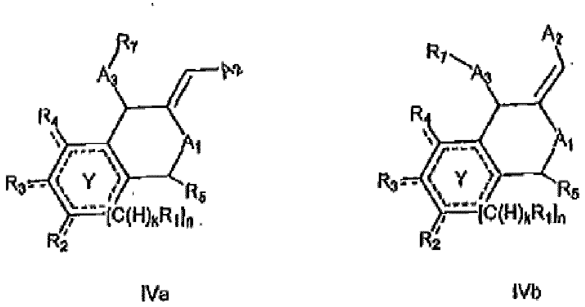 wobei die Definitionen von Y, A₁, A₂, A₃, R₁, R₂, R₃, R₄, R₅, R₆, R₇, k und n die gleichen wie diejenigen von Anspruch 1 oder 2 sind.

4. Neuartige antibiotische und Antikrebs-Verbindung mit der allgemeinen Formel I, II, III oder IV, Derivate, Stereoisomere, die racemische oder nicht racemische Mischung der Stereoisomere oder das pharmazeutisch akzeptable Salz oder Solvat der Verbindungen nach Anspruch 1, wobei neue antibakterielle Antikrebs-Verbindungen, die durch die Verbindungen mit der Formel I, II, III oder IV dargestellt sind, Folgendes umfassen:
(Z)-6-chlor-3-(chlormethylen)thiochroman-4-on
(Z)-3-(chlormethylen)-6-methylthiochroman-4-on
(Z)-3-(chlormethylen)-7-fluor-6-methylthiochroman-4-on
(Z)-3-(chlormethylen)-6-fluorthiochroman-4-on
(Z)-3-(brommethylen)-6-methylthiochroman-4-on
(Z)-3-(brommethylen)-7-fluor-6-methylthiochroman-4-on
(Z)-3-(brommethylen)-6-chlorthiochroman-4-on
(Z)-3-(brommethylen)-6-fluorthiochroman-4-on
(Z)-6-fluor-3(((3-fluor-4-methylphenyl)thio)methylen)-thiochroman-4-on
(Z)-3-(chlormethylen)-isothiochroman-4-on
(Z)-3-(chlormethylen)-6-fluorisothiochroman-4-on
(Z)-3-(brommethylen)-7-fluor-6-methylisothiochroman-4-on
(Z)-3-(chlormethylen)-6-methylthiochroman-4-ol
(Z)-3-(chlormethylen)-2H-thiopyrano[2,3-b]pyridin-4(3H)-on
(Z)-5-(chlormethylen)-5,6-dihydro-4H-thieno[2,3-b]thiopyran-4-on
(Z)-3-(chlormethylen)-isothiochroman-4-ol
(E)-6-chlor-3(chlormethylen)thiochroman-4-on
(E)-3-(chlormethylen)-6-fluor-thiochroman-4-on
(E)-6-fluor-3(((3-fluor-4-methylphenyl)thio)methylen)-thiochroman-4-on
(E)-5-(chlormethylen)-5,6-dihydro-4H-thieno[2,3-b]thiopyran-4-on
(E)-3-(chlormethylen)-6-methylthiochroman-4-ol
(E)-3-(chlormethylen)-isothiochroman-4-on
(E)-3-(chlormethylen)-2H-thiopyrano[2,3-b]pyridin-4(3H)-on
(E)-4-chlor-3(chlormethylen)-6-methylthiochroman
(E)-3-(chlormethylen)-6-methyl-4-(p-tolylthio)thiohroman

5. Zubereitungsverfahren der neuartigen antibiotischen und Antikrebs-Verbindung mit der allgemeinen Formel I, II, III oder IV, Derivate, Stereoisomere, die racemische oder nicht racemische Mischung der Stereoisomere oder das pharmazeutisch akzeptable Salz oder Solvat der Verbindungen, die Folgendes umfassen:
1) die Verbindung VIII wird durch die Reaktion von Verbindung V mit Verbindung VII und Base 1 zubereitet; die Verbindung IX wird durch die Reaktion von Verbindung VI mit Verbindung VII und Base 1 zubereitet. 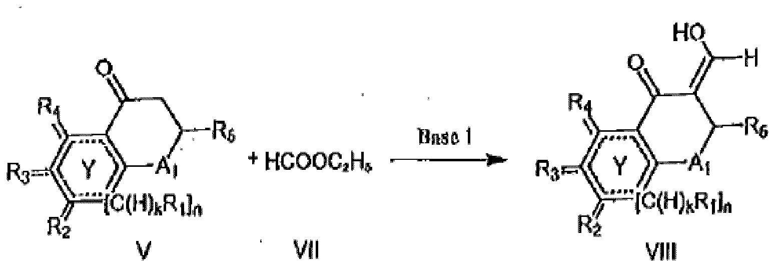 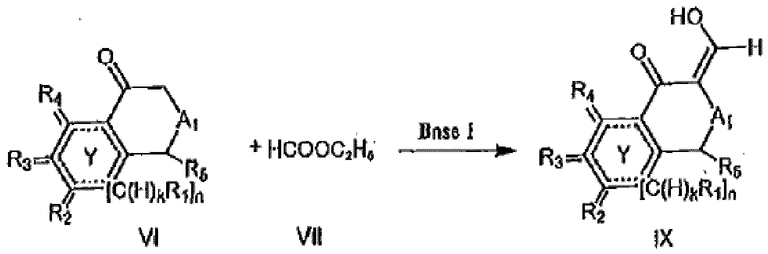
2) die Cis- und Transisomere von den Verbindungen XIa und XIb werden durch die Reaktion von Verbindung VIII mit Verbindung X' zubereitet, und die Cis- und Transisomere von den Verbindungen XIIa und XIIb werden durch die Reaktion von Verbindungen IX mit Verbindung X' zubereitet, 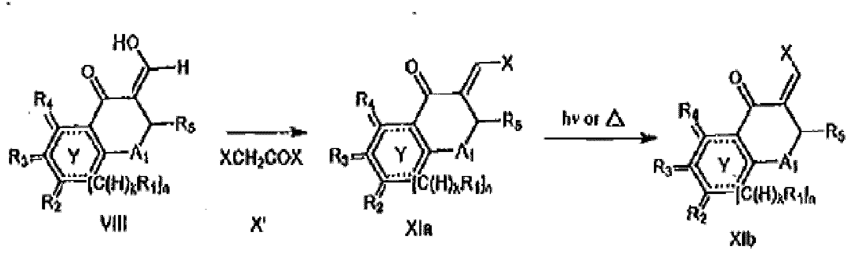 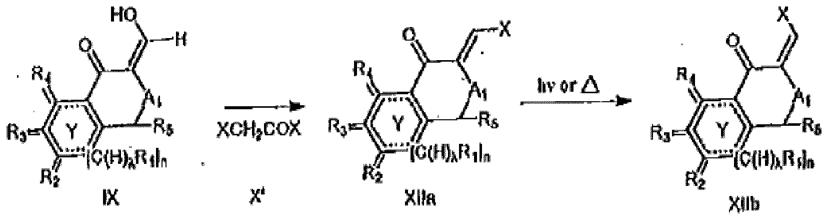
3) die Verbindung XVII wird durch die Reaktion von Verbindung XIV mit Verbindung XVI und Katalysator 1 zubereitet, und die Verbindung XVIII wird durch die Reaktion von Verbindung XV mit Verbindung XVI und Katalysator 1 zubereitet; 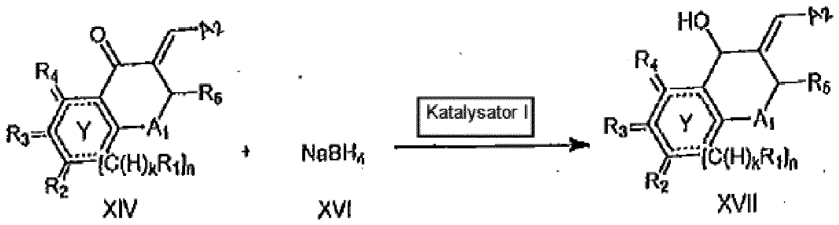 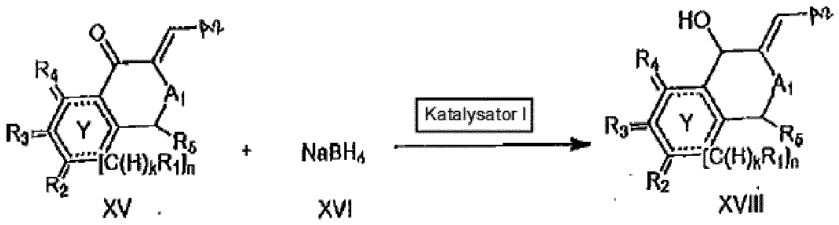
4) die Verbindung XIX wird durch die Reaktion von Verbindung XVII mit einem Halogenierungsreagens zubereitet, und die Verbindung XX wird durch die Reaktion von Verbindung XVIII mit einem Halogenierungsreagens zubereitet; 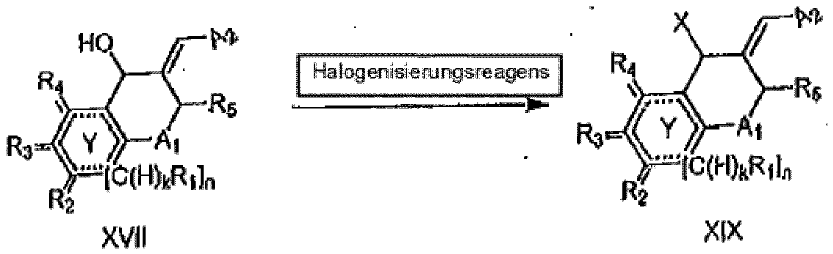 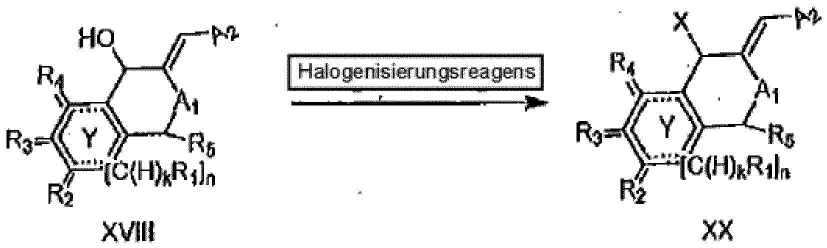
5) die Verbindung XXII wird durch die Reaktion von Verbindung XIX mit Verbindung XXI und Base 2 zubereitet, und die Verbindung XXIII wird durch die Reaktion von Verbindung XX mit Verbindung XXI und Base 2 zubereitet. 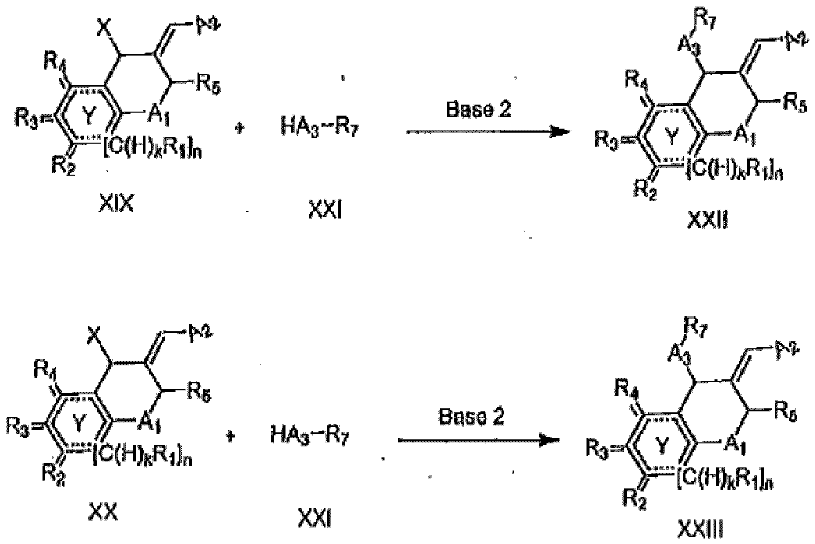
Die Transisomere der Verbindungen XIV, XV, XVII, XVIII, XIX und XX können wie die Verfahren ihrer Cisisomerzubereitung zubereitet werden, und die Transisomere der Verbindung XVII, VIII, IX (XX), (XXII) und XXIII können auf den oben erwähnten Wegen zubereitet werden;
wobei Y, A₁, A₂, A₃, R₁, R₂, R₃, R₄, R₅, R₇, k, n und die Definition der gepunkteten Linie die gleiche wie diejenige von Anspruch 1 oder 2 sind; X ist Halogen.

6. Medizinische Kombination einer neuartigen antibiotischen oder Antikrebs-Verbindung mit der allgemeinen Formel I, II, III oder IV, Derivate, Stereoisomere, die racemische oder nicht racemische Mischung der Stereoisomere oder das pharmazeutisch akzeptable Salz oder Solvat der Verbindungen nach Anspruch 1, 2, 3, 4 oder 5, pharmazeutische Hilfsmittel oder optional ein pharmazeutischer Trägerstoff.

7. Medizinische Kombination nach Anspruch 6, wobei die Dosierungsform der medizinischen Kombination die Zubereitungsform Salbe, Kremor, Gelee, Creme, Lotion, Uppos, Öle, massa pitularum, Tabletten, Collocystis, injizierbare Zubereitung und Conspergens annehmen kann.

8. Verbindung mit der allgemeinen Formel I, II, III oder IV, Derivate, Stereoisomere, die racemische oder nicht racemische Mischung der Stereoisomere oder das pharmazeutisch akzeptable Salz oder Solvat der Verbindungen nach Anspruch 1, 2, 4 oder 4 zur Verwendung als antibiotisches und Antikrebs-Arzneimittel, bei der Behandlung oder Prävention von Infektionen, verursacht von Bakterien, wobei das Wachstum von kanzerösen Tumorzellen oder damit assoziierten Krankheiten bei einem Wirbeltier verhindert wird.

9. Verwendung von Anspruch 8, wobei die Bakterien blastomyces albicans, candida tropicalis, Backhefe, cryptococcus neoformans, aerothesium floccosum, trichophyton gypseum, trichophyton rubrum, trichophyton tonsurans, microsporum gypseum, trichoderma, aspergilius niger, A. glaucus, penicillun commune, fehldiagnostizierter Fonsecaea-Pedrosoi, cladosporium carrionii, phialophora compacta, phialophora verrucosa, sporothrix schenckii, Staphylococcus aureus, bacillus coli, Erythromycin-Schimmel, Großblock-Borstenhohlraum-Fleckpilz und Fusarium-Pilze et al umfassen, wobei die Krebszellen Magenkrebs, Darmkrebs, Leberkrebs, Bauchspeicheldrüsenkrebs, Speiseröhrenkrebs B01092, chondroma sarcomatosum, Melanom, Hodkin'sche Krankheit, Leukämie, Brustkrebs, Prostatakrebs, Schilddrüsenkrebs, Hautkrebs und Blasenkrebs usw. umfassen.

10. Verwendung der neuartigen antibiotischen und Antikrebs-Verbindung mit der allgemeinen Formel I, II, III oder IV, Derivate, Stereoisomere, die racemische oder nicht racemische Mischung der Stereoisomere oder das pharmazeutisch akzeptable Salz oder Solvat der Verbindung nach Anspruch 1, 2, 3 oder 4, zur Zubereitung von Arzneimittel, das verwendet werden kann, um eine Infektion, verursacht von Bakterien, zu behandeln oder vorzubeugen, um das Wachstum von kanzerösen Tumorzellen und damit assoziierten Krankheiten bei einem Säugetier zu verhindern.

## Revendications

1. Nouveau composé antibiotique et anti-cancéreux de formule générale I, II, III ou IV, dérivés, stéréoisomères, mélange racémique ou non-racémique des stéréoisomères ou sel ou solvate pharmaceutiquement acceptable des composés, la formule générale étant indiquée comme suit : 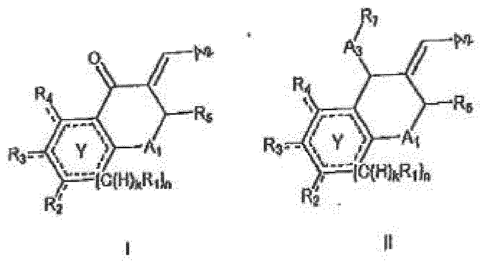 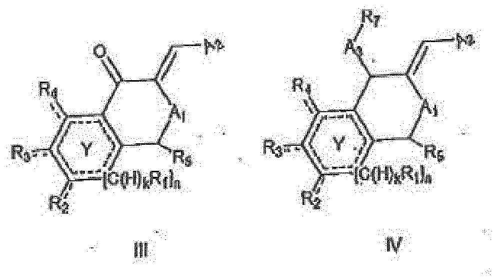
dans lesquels Y représente les éléments du cycle benzène dans n'importe quelle position, et il peut être indépendamment choisi parmi un C, O, S et N ; lorsque Y représente un O ou S, c'est un élément bivalent ; Y est un élément trivalent quand il représente N ; et c'est un élément quadrivalent quand il représente un C, Y est de préférence choisi parmi un C, N ou S ;
les lignes en pointillés représentent les liaisons qui sont superflues, lorsqu'une liaison est une double liaison, ses liaisons voisines ne sont pas des doubles liaisons ;
k est un nombre entier 0 ou 1 ; n est un nombre entier 0, 1 ou 2 ;
R₁, R₂, R₃ et R₄ peuvent être indépendamment choisis parmi un hydrogène, un fluor, un chlore, un brome, un iode, un hydroxyle, un cyano, un alkyle en C₁₋₂₀, un alkyle-oxy en C₁₋₂₀, un alkyl-carbonyle en C₁₋₂₀ et un alkyl-carbonyle-oxy en C₁₋₂₀, à la condition qu'au moins un élément parmi R₁, R₂, R₃ et R₄ ne soit pas un hydrogène et la partie alkyle dans ces groupes peut être remplacée par un ou plusieurs des agents d'halogène indépendants comme le fluor, le chlore, le brome et l'iode ;
R₅ représente un hydrogène, un alkyle en C₁₋₂₀, un alkyl-oxy en C₁₋₂₀, un alkyl-carbonyle en C₁₋₂₀ ou un alkyl-carbonyl-oxy en C₁₋₂₀ ;
R₇ représente un hydrogène, un alkyle, un aryle, un aryle substitué ou un hétéroaryle ;
A₁ représente un CH₂, un CH₂CH₂, un O, S, S(O), S(O)₂ ou NR₁ ;
A₂ représente un Cl, Br, F ou I ;
A₃ représente un O, S, S(O), S(O)₂, NR, Cl, Br, F, I ou P ; quand A₃ est un Cl, Br, F ou I, R₇ n'existe pas ;
dans lesquels un hydrocarbonyle en C₁₋₂₀ est un groupe hydrocarbure aromatique ou un hydrocarbonyle non-aromatique, un hydrocarbonyle à chaîne droite ou un hydrocarbonyle à chaîne ramifiée, un hydrocarbonyle cyclique ou un hydrocarbonyle non-cyclique.

2. Nouveau composé antibiotique et anti-cancéreux de formule générale I, II, III ou IV, dérivés, stéréoisomères, mélange racémique ou non-racémique des stéréoisomères, ou sel ou solvate pharmaceutiquement acceptable du composé selon la revendication 1, dans lequel l'hydrocarbonyle en C₁₋₂₀ est choisi parmi un alkyle en C₁₋₂₀, un alcényle en C₂₋₂₀, un alcynyle en C₂₋₂₀, un cycloalcanyle en C₃₋₂₀, un cycloalcényle en C₃₋₂₀, un aryle en C₆₋₂₀, un aryle en C₆₋₂₀-alkyle en C₁₋₁₀, un cycloalcanyle en C₃₋₁₀-alkyle en C₁₋₁₀, un cycloalcényle en C₃₋₁₀-alkyle en C₁₋₁₀ et un alkyle en C₁₋₁₀-aryle en C₆₋₁₀.

3. Nouveau composé antibiotique et anti-cancéreux de formule générale I, II, III ou IV, dérivés, stéréoisomères, mélange racémique ou non-racémique des stéréoisomères, ou sel ou solvate pharmaceutiquement acceptable du composé selon la revendication 1 ou 2, dans lequel le composé I est le stéréoisomère ou mélange de composé Ia et Ib ; le composé II est le stéréoisomère ou mélange de composé IIa et IIb ; le composé III est le stéréoisomère ou mélange de composé IIIa et IIIb, le composé IV est le stéréoisomère ou mélange de composés IVa et IVb. 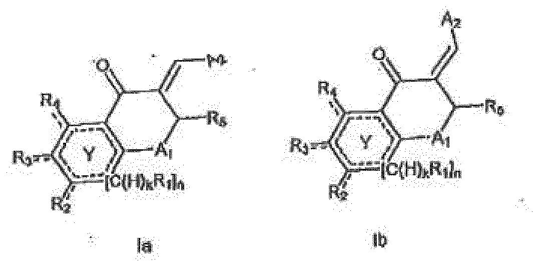 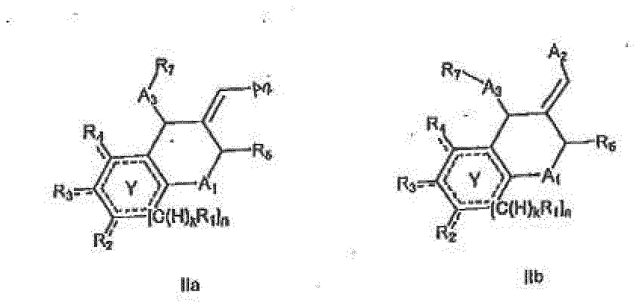 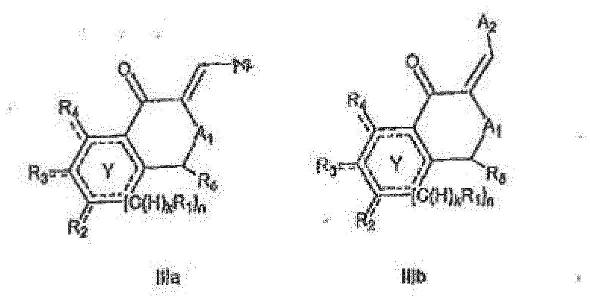 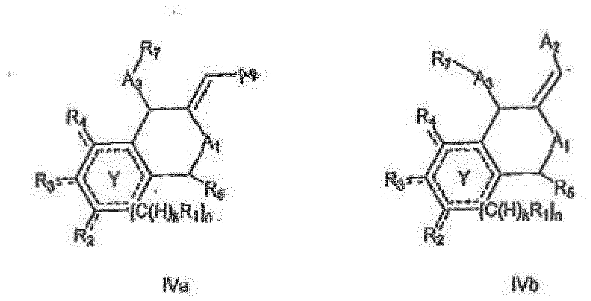 dans lesquels les définitions de Y, A₁, A₂, A₃, R₁, R₂, R₃, R₄, R₅, R₆, R₇, k et n sont identiques à celles de la revendication 1 ou 2.

4. Nouveau composé antibiotique et anti-cancéreux de formule générale I, II, III ou IV, dérivés, stéréoisomères, mélange racémique ou non-racémique des stéréoisomères, ou sel ou solvate pharmaceutiquement acceptable du composé selon la revendication 1, dans lequel de nouveaux composés antibactériens et anti-cancéreux qui sont représentés par les composés de formule I, II, III ou IV comprennent les composés suivants :
(Z)-6-chloro-3-(chlorométhylène)thiochroman-4-one
(Z)-3-(chlorométhylène)-6-méthylthiochroman-4-one
(Z)-3-(chlorométhylène)-7-fluoro-6-méthylthiochroman-4-one
(2)-3-(chlorométhylène)-6-fluorothiochroman-4-one
(Z)-3-(bromométhylène)-6-méthylthiochroman-4-one
(Z)-3-(bromométhylène)-7-fluoro-6-méthylthiochroman-4-one
(Z)-3-(bromométhylène)-6-chlorothiochroman-4-one
(Z)-3-(bromométhylène)-6-fluorothiochroman-4-one
(Z)-6-fluoro-3-(((3-fluoro-4-méthylphényl)-thio)méthylène)thiochroman-4-one
(Z)-3-(chlorométhylène)isothiochroman-4-one
(Z)-3-(chlorométhylène)-6-fluoroisothio-chroman-4-one
(Z)-3-(bromométhylène)-7-fluoro-6-méthyliso-thiochroman-4-one
(Z)-3-(chlorométhylène)-6-méthylthiochroman-4-ol
(Z)-3-(chlorométhylène)-2H-thiopyrano[2,3-h]pyridine-4(3H)-one
(Z)-5-(chlorométhylène)-5,6-dihydro-4H-thiéno[2,3-b]thiopyran-4-one
(Z)-3-(chlorométhylène)isothiochroman-4-ol
(E)-6-chloro-3-(chlorométhylène)thiochroman-4-one
(E)-3-(chlorométhylène)-6-fluorothiochroman-4-one
(E)-6-fluoro-3(((3-fluoro-4-méthylphényl)-thio)méthylène)thiochroman-4-one
(E)-5-(chromométhylène)-5,6-dihydro-4H-thiéno[2,3-b]thiopyran-4-one
(E)-3-(chlorométhylène)-6-méthylthiochroman-4-ol
(E)-3-(chlorométhylène)isothiochroman-4-one
(E)-3-(chlorométhylène)-2H-thiopyrano[2,3-b]pyridin-4(3H)-one
(E)-4-chloro-3-(chlorométhylène)-6-méthylthio-chromane
(E)-3-(chlorométhylène)-6-méthyl-d-(p-tolylthio)thiochromane

5. Procédé de préparation dudit nouveau composé antibiotique et anti-cancéreux de formule générale I, II, III ou IV, dérivés, stéréoisomères, mélange racémique ou non-racémique des stéréoisomères, ou sel ou solvate pharmaceutiquement acceptable du composé, comprenant les éléments suivants :
1) le composé VIII est préparé par la réaction de composés V avec le composé VII et la Base 1 : le composé IX est préparé par la réaction de composés VI avec le composé VII et la Base 1. 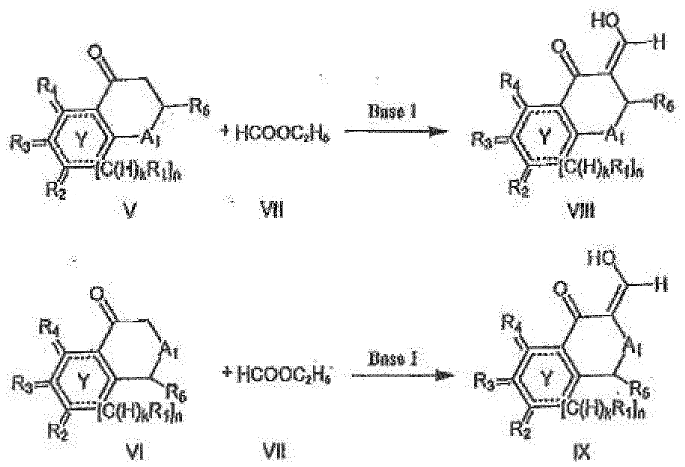
2) les isomères cis et trans de composés XIa et XIb sont préparés par la réaction de composés VIII avec le composé X' et les isomères cis et trans de composés XIIa et XIIb sont préparés par la réaction de composés IX avec le composé X' ; 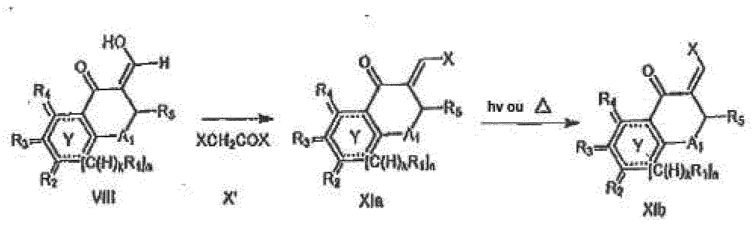 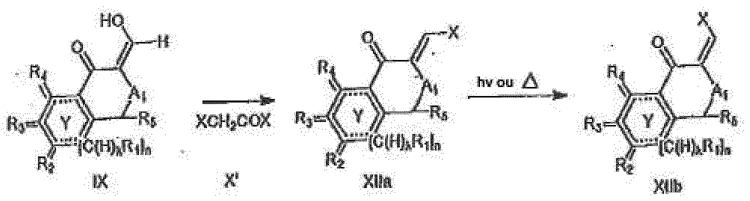
3) le composé XVII est préparé par la réaction du composé XIV avec le composé XVI et le catalyseur I, et le composé XVIII est préparé par la réaction du composé XV avec le composé XVI et le catalyseur 1 ; 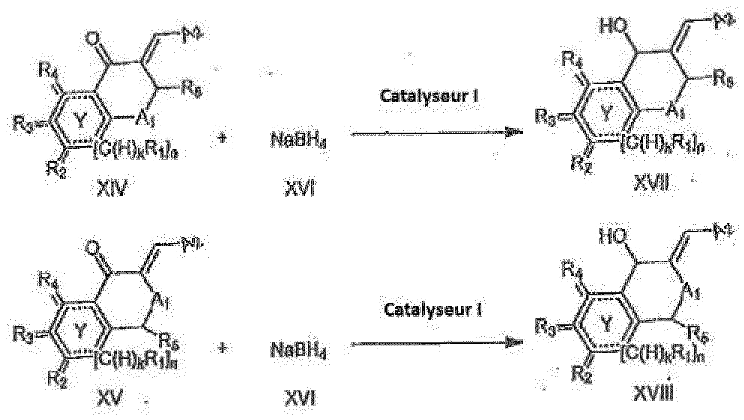
4) le composé XIX est préparé par la réaction du composé XVII avec un réactif d'halogénation et le composé XX est préparé par la réaction du composé XVIII avec un réactif d'halogénation ; 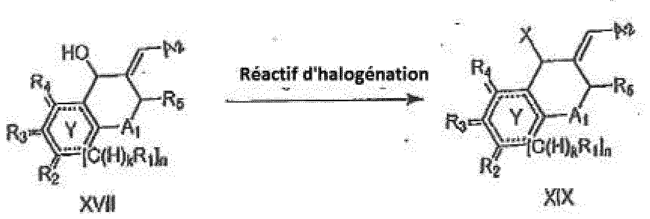 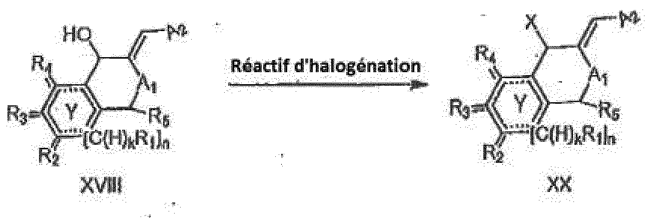
5) le composé XXII est préparé par la réaction du composé XIX avec le composé XXI et la base 2, et le composé XXIII est préparé par la réaction de composé XX avec le composé XXI et la base 2 ; 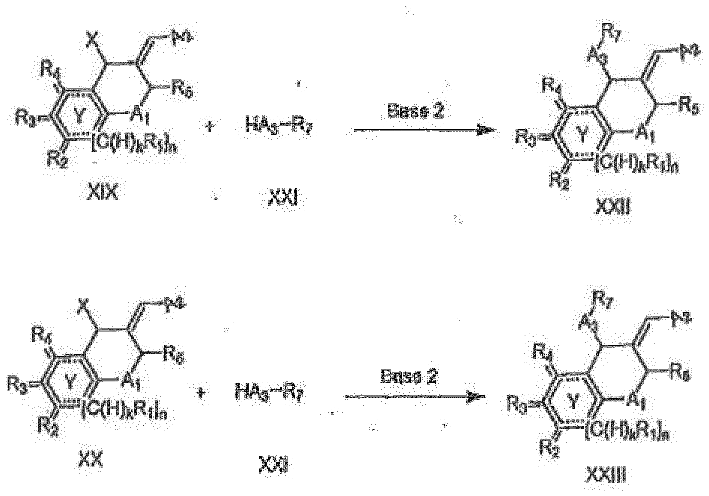
des trans-isomères de composés XIV, XV, XVII, XVIII, XIX et XX peuvent être préparés comme les procédés de leur préparation cis-isomères, et des trans-isomères de composé XVII, VIII, IX (XX), (XXII) et XXIII peuvent être obtenus des façons mentionnées ci-dessus ;
dans lesquels Y, A₁, A₂, A₃, R₁, R₂, R₃, R₄, R₅, R₇, k, n et la définition de la ligne pointillée sont identiques à celles de la revendication 1 ou 2 ; X est un halogène.

6. Combinaison médicinale de nouveau composé antibiotique et anti-cancéreux de formule générale I, II, III ou IV, dérivés, de stéréoisomères, de mélange racémique ou non-racémique des stéréoisomères, ou de sel ou solvate pharmaceutiquement acceptable du composé selon la revendication 1, 2, 3, 4 ou 5, et d'adjuvants pharmaceutiques ou d'un excipient pharmaceutique éventuel.

7. Combinaison médicinale selon la revendication 6, dans laquelle la forme de dosage de la combinaison médicinale peut être une préparation sous forme de pommade, de crème, de gelée, de lotion, de suppositoires, d'huiles, de *massa pilularum,* de comprimés, de collocystis, de préparation injectable et d'agent mouillant.

8. Composé de formule générale I, II, III ou IV, dérivés, stéréoisomères, mélange racémique ou non-racémique des stéréoisomères, ou sel ou solvate pharmaceutiquement acceptable du composé selon la revendication 1, 2, 3, ou 4 à utiliser comme médicament antibiotique et anticancéreux dans le traitement ou la prévention d'une infection provoquée par des bactéries, en inhibant la croissance de cellules de tumeurs cancéreuses ou de maladies associées chez un mammifère.

9. Composé selon la revendication 8, dans laquelle lesdites bactéries comprennent des blastomyces albicans, des candida tropicalis, des levures de boulangers, des cryptococcus neoformans, des aerothesium floccosum, des trichophyton gypseum, des trichophyton rubrum, des trichophyton tonsurans, des microsporum gypseum, des trichoderma, des aspergillus niger, des A. glaucus, des penicillum commune, des Fonsecaea-Pedrosoi mal diagnostiqués, des cladosporium carrionii, des phialophora compacta, des phialophora verrucosa, des sporothrix schenckii, des staphylococcus aureus, des bacillus coli, des érythro-moisissure, des champignons à cavité filamenteuse à gros bloc et des fosarium fungi, entre autres, lesdites cellules cancéreuses comprenant un cancer gastrique, un cancer des intestins, un cancer du foie, un cancer du pancréas, un cancer de l'oesophage B01092, un chondroma sarcomatosum, un mélanome, une maladie de Hodgkin, une leucémie, un cancer du sein, un carcinome de la prostate, un cancer de la thyroïde, un cancer de la peau et un carcinome de la vessie, etc.

10. Utilisation dudit nouveau composé antibiotique et anti-cancéreux de formule générale I, II, III ou IV, dérivés, stéréoisomères, mélange racémique ou non-racémique des stéréoisomères, ou sel ou solvate pharmaceutiquement acceptable du composé selon la revendication 1, 2, 3, ou 4 pour préparer un médicament pouvant être utilisé pour traiter ou prévenir l'infection provoquée par les bactéries, pour inhiber la croissance de cellules de tumeurs cancéreuses et des maladies associées chez un mammifère.
